# EUROPEAN PATENT SPECIFICATION

(11) **EP 1 651 220 B1**
(45) Date of publication and mention of the grant of the patent: **20.12.2006**
(21) Application number: 04741322.4
(22) Date of filing: 29.07.2004
(51) Int. Cl.: A61K 31/454, A61P 3/04

(54) **2-[4- (2-HYDROXYMETHYL-PHENYLAMINO)-PIPERIDIN-1-YL] - N-(9H-CARBAZOL-3-YL) - ACETAMIDE DERIVATIVES AND RELATED COMPOUNDS AS NEUROPEPTIDE Y5 (NPY5) LIGANDS FOR THE TREATMENT OF OBESITY**
2-[4-(2-HYDROXYMETHYL-PHENYLAMINO)-PIPERIDIN-1-YL]-N-(9H-CARBAZOL-3-YL) - ACETAMID DERIVATE UND VERWANDTE VERBINDUNGEN ALS NEUROPEPTIDE Y5 (NPY5) LIGANDEN ZUR BEHANDLUNG VON FETTLEIBIGKEIT
DERIVES DE 2- [4- (2-HYDROXYMETHYL-PHENYLAMINO) -PIPERIDINE-1-YL]- N- (9H-CARBAZOL-3-YL) - ACETAMINE ET DES COMPOSES ASSOCIES EN TANT QUE LIGANDS DE NEUROPEPTIDE Y5 (NPY5) POUR LE TRAITEMENT DE L'OBESITE

(30) Priority: 30.07.2003 ES 200301813
(43) Date of publication of application: 03.05.2006
(73) Proprietor: LABORATORIOS DEL DR. ESTEVE, S.A., 08041 Barcelona (ES)
(72) Inventor: TORRENS JOVER, Antoni, E-08221 Barcelona (ES); MAS PRIO, Josep, E-08191 Rubi, Barcelona (ES); DORDAL ZUERAS, Alberto, E-08016 Barcelona (ES); FISAS ESCASANY, Maria Angeles, E-08025 Barcelona (ES); BUSCHMANN, Helmut, Heinrich, E-08950 Esplugues de Llobregat (ES)
(74) Representative: Carvajal y Urquijo, Isabel
(86) International application number: PCT/EP2004/008517
(87) International publication number: WO 2005/013990

(56) References cited:
- WO-A-98/35957
- WO-A-03/084939
- US-B1- 6 399 631

## Description

The present invention relates to 1,4-disubstituted piperidine compounds of general formula (I), methods for their preparation, medicaments comprising these compounds as well as their use for the preparation of a medicament for the treatment of humans or animals.

Neuropeptide Y (NPY), first isolated in porcine brain extracts (Tatemoto et. al. Nature 1982, 296, 659), is a 36-aminoacid peptide belonging to the family of pancreatic polypeptides, and is one of the most abundant peptides in the brain and in the central nervous system. Several studies suggest that NPY plays a significant role in cognitive function regulation, e. g. memory (Flood J. F. et. al. Brain Res. 1987, 421, 280; Redrobe J. P. et. Al. Brain Res. 1999, 848, 153), and in the development of anxiety (Heilig M. et. al. Reg. Peptides 1992, 41, 61) and depression (Heilig M. et. al. Eur. J. Pharmacol. 1988, 147, 465).

Moreover, NPY is also distributed in the peripheral system and some studies suggest that it is involved in hypertensive (Michel M. C: et. al. J. Hypertens. 1995, 13, 385), and analgesic (Gehlert D. R. Life Sci. 1994, 55, 551) processes, among others.

The endogenous proteins that constitute NPY-binding receptors have been widely studied. Several have been cloned and expressed. At present, six different receptor subtypes, named Y1 to Y6, are recognized (Hispkind P. A. et. al. Annu. Rep. Med. Chem. 1996, 31, 1; Grundemar L. et. al. TIPS Reviews., 15, 153, 1994). Each NPY receptor subtype is generally associated to a different biological activity.

The most recently identified receptor is Y5 (Hu et. al. J. Biol. Chem. 1996, 271, 26315). Since there is ample evidence that the Y5 receptor has a unique pharmacological profile compared to the other receptor subtypes, it was initially expected that the Y5 receptor might be a suitable target for the treatment of food intake related disorders such as obesity. This attitude has changed and it is now the common opinion among those skilled in the art that the Y5 receptor in general is not a suitable target for a treatment of food intake related disorders such as obesity. This change in attitude may be attributed to the fact that the compounds with affinity for the NPY-5 receptor tested so far were not orally active, see also Current Opinion in Investigational Drugs 2003, 4, 1198; Diabetes Vol. 51, August 2002, 2441 and International Journal of Obesity 2004, 28, 628.

Thus, it was an object of the present invention to provide novel compounds that are suitable in particular as active substances in medicaments, preferably in medicaments for the regulation of neuropeptide Y receptors, particularly preferably of neuropeptide Y 5 (NPY5) receptor, for the regulation of food intake and for the prophylaxis and/or treatment of food intake related disorders such as obesity, anorexia, cachexia, bulimia or type II (non insulin dependent) diabetes.

Surprisingly, it has been found that the 1,4-disubstituted piperidine compounds of general formula (I) given below have affinity for neuropeptide Y receptors, in particular for neuropeptide Y 5 (NPY5) receptors. Moreover, the compounds according to the present invention have surprisingly been found to show significant appetite suppressing effects.

Therefore, in one of its aspects the present invention relates to 1,4-disubstituted piperidine compounds of general formula (I), 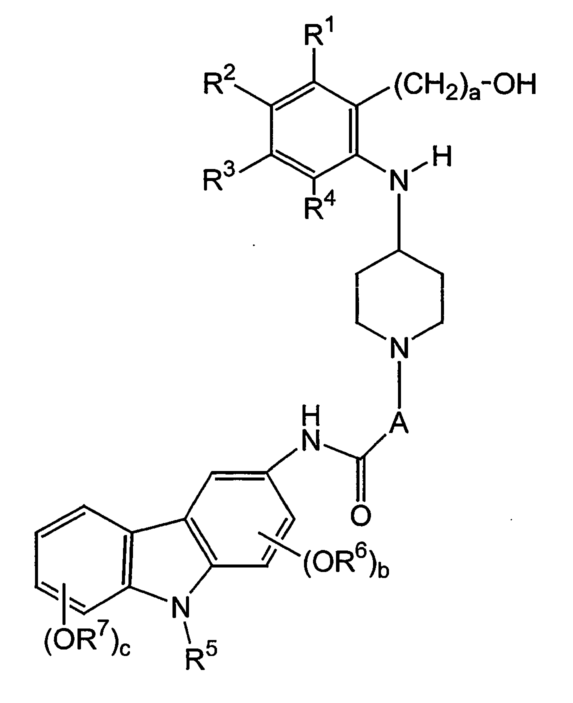 wherein
a represents 0, 1, 2, 3 or 4,
b represents 0, 1, 2 or 3,
c represents 0, 1, 2, 3 or 4,
R¹, R², R³, R⁴ are each independently selected from the group consisting of hydrogen; halogen; -CN; -NO₂; -OR⁸; a linear or branched, saturated or unsaturated, optionally at least mono-substituted aliphatic radical; a saturated or unsaturated, optionally at least mono-substituted, optionally at least one heteroatom as ring member containing cycloaliphatic radical, which may be bonded via an alkylene group; or an optionally at least mono-substituted aryl- or heteroaryl radical, which may be bonded via an alkylene group and/or which may be condensed with an optionally at least mono-substituted, saturated or unsaturated mono- or bicyclic ring system,
R⁵ represents hydrogen, a linear or branched, saturated or unsaturated, optionally at least mono-substituted aliphatic radical, or a saturated or unsaturated, optionally at least mono-substituted cycloaliphatic radical,
R⁶, R⁷ and R⁸, identical or different, each represent hydrogen or a prodrug-moiety,
A represents a -CH₂- or -CH₂-CH₂- group,
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

Preferred are 1,4-disubstituted piperidine compounds of general formula (I) given above, wherein
a represents 0, 1, 2, 3 or 4,
b represents 0, 1, 2 or 3,
c represents 0, 1, 2, 3 or 4,
R¹, R², R³, R⁴ are each independently selected from the group consisting of H; F; Cl; Br; -CN; -NO₂; -OR⁸; a linear or branched, saturated or unsaturated, optionally at least mono-substituted C₁₋₆-aliphatic radical; a saturated or unsaturated, optionally at least mono-substituted, optionally at least one heteroatom as ring member containing C₃₋₈-cycloaliphatic radical, which may be bonded via a C₁₋₃-alkylene group; or an optionally at least mono-substituted aryl- or heteroaryl radical, which may be bonded via a C₁₋₃-alkylene group and/or which may be condensed with an optionally at least mono-substituted, saturated or unsaturated mono- or bicyclic ring system,
R⁵ represents hydrogen, a linear or branched, saturated or unsaturated, optionally at least mono-substituted C₁₋₆-aliphatic radical, or a saturated or unsaturated, optionally at least mono-substituted C₃₋₈-cycloaliphatic radical,
R⁶, R⁷ and R⁸, identical or different, each represent hydrogen or a prodrug-moiety,
A represents a -CH₂- or -CH₂-CH₂- group,
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

A prodrug moiety in the sense of the present invention is a moiety that will give rise to a pharmacologically active metabolite of the respective compound of general formula I in vivo.

Suitable prodrug moieties, methods for their preparation, methods for their introduction into a starting compound to give a compound of general formula I as defined above as well as methods for determination of the metabolite formed in vivo are well known to those skilled in the art, e.g. from the textbooks of Krogsgaard-Larsen, Povl, "A textbook of drug design and development" Harwood Academic (ISBN 3-7186-5100-9) and from Bernard Testa and Joachim B. Mayer, "Hydrolysis in drug and prodrug metabolism: Chemistry, biochemistry and enzymology, Wiley-VCH, 2003, Weinheim (ISBN-3-906390-25-X). The respective parts of the literature description are hereby incorporated by reference and form part of the present disclosure.

A mono- or bicyclic ring system according to the present invention means a mono- or bicyclic hydrocarbon ring system that may be saturated, unsaturated or aromatic. If the ring system is bicyclic, each of its different rings may show a different degree of saturation, i.e. it may be saturated, unsaturated or aromatic. Optionally each of the rings of the mono- or bicyclic ring system may contain one or more heteroatoms as ring members, which may be identical or different and which can preferably be selected from the group consisting of N, O, S and P, more preferably be selected from the group consisting of N, O and S. Said mono- or bicyclic ring-system may preferably contain 0, 1, 2 or 3 heteroatoms chosen from the afore mentioned group, preferably it contains 0 or 1 heteroatoms chosen from the afore mentioned group. The rings of the mono- or bicyclic ring system are preferably 5- or 6-membered.

Those skilled in the art understand that the term "condensed" indicates that the condensed rings share more than one atom. The terms "annulated" or "fused" may also be used for this type of bonding.

If one or more of the residues R¹-R⁵ represents an aliphatic radical, which is substituted by one or more substituents, unless defined otherwise, each of these substituents may preferably be selected from the group consisting of hydroxy, halogen, branched or linear C₁₋₄-alkoxy, branched or linear C₁₋₄-perfluoroalkoxy, branched or linear C₁₋₄-perfluoroalkyl, amino, carboxy, amido, cyano, nitro, -SO₂NH₂, -CO-C₁₋₄-alkyl, -SO-C₁₋₄-alkyl, -SO₂-C₁₋₄-alkyl, -NH-SO₂-C₁₋₄-alkyl , wherein the C₁₋₄-alkyl may in each case be branched or linear, an unsubstituted or at least mono-substituted phenyl or naphthyl radical and an unsubstituted or at least mono-substituted furanyl-, thienyl-, pyrrolyl-, imidazolyl-, pyrazolyl-, pyridinyl-, pyrimidinyl-, quinolinyl- and isoquinolinyl radical, more preferably be selected from the group consisting of hydroxy, F, Cl, Br, methoxy, ethoxy, CF₃ and an unsubstituted phenyl radical. If any one of these substituents itself is at least mono-substituted, said substituents may preferably be selected from the group consisting of F, Cl, methyl and methoxy. Preferably the substituted alkyl radical may be substituted with 1, 2, 3, 4 or 5, more preferably with 1, 2 or 3 of the afore mentioned substituents.

If one or more of the residues R¹-R⁵ represents or comprises a cycloaliphatic radical, which is substituted by one or more substituents, unless defined otherwise, each of these substituents may preferably be selected from the group consisting of hydroxy, halogen, branched or linear C₁₋₄-alkyl, branched or linear C₁₋₄-alkoxy, branched or linear C₁₋₄-perfluoroalkoxy, phenoxy, benzoyl, cyclohexyl, branched or linear C₁₋₄-perfluoroalkyl, -NR^{A}R^{B} wherein R^{A}, R^{B} are each independently selected from the group consisting of H, a branched or linear C₁₋₄-alkyl-radical, -CH₂-CH₂-OH and phenyl, carboxy, amido, cyano, nitro, -SO₂NH₂, - CO-C₁₋₄-alkyl, -CO-OC₁₋₄-alkyl, -SO-C₁₋₄-alkyl, -SO₂-C₁₋₄-alkyl, -NH-SO₂-C₁₋₄-alkyl, wherein C₁₋₄-alkyl may in each case be branched or linear, unsubstituted or at least mono-substituted phenyl or naphthyl and an unsubstituted or at least mono-substituted furanyl-, thienyl-, pyrrolyl-, imidazolyl-, pyrazolyl-, pyridinyl-, pyrimidinyl-, quinolinyl- and isoquinolinyl radical, more preferably be selected from the group consisting of hydroxy, F, Cl, Br, methyl, ethyl, methoxy, ethoxy, benzoyl, phenoxy, cyclohexyl, -CF₃, -CO-CH₃, -CO-OCH₃, -NR^{A}R^{B} wherein R^{A}, R^{B} are each independently selected from the group consisting of H, a branched or linear C₁₋₄-alkyl-radical, -CH₂-CH₂-OH and phenyl, and an unsubstituted phenyl radical. If any one of these substituents itself is at least mono-substituted, said substituents may preferably be selected from the group consisting of F, Cl, methyl and methoxy. Preferably the substituted cycloaliphatic radical may be substituted with 1, 2, 3, 4 or 5, more preferably with 1, 2 or 3 of the afore mentioned substituents.

If one or more of the residues R¹-R⁴ comprises a mono- or bicycyclic ring system, which is substituted by one or more substituents, unless defined otherwise, each of these substituents may preferably be selected from the group consisting of hydroxy, halogen, branched or linear C₁₋₄-alkyl, branched or linear C₁₋₄-alkoxy, branched or linear C₁₋₄-perfluoroalkoxy, branched or linear C₁₋₄-perfluoroalkyl, amino, carboxy, amido, cyano, keto, nitro, -SO₂NH₂, -CO-C₁₋₄-alkyl, -SO-C₁₋₄-alkyl, -SO₂-C₁₋₄-alkyl, -NH-SO₂-C₁₋₄-alkyl, wherein C₁₋₄-alkyl may be branched or linear, an unsubstituted or at least mono-substituted phenyl or naphthyl radical and unsubstituted or at least mono-substituted furanyl-, thienyl-, pyrrolyl-, imidazolyl-, pyrazolyl-, pyridinyl-, pyrimidinyl-, quinolinyl- and isoquinolinyl, more preferably from the group consisting of hydroxy, F, Cl, Br, methyl, ethyl, methoxy, ethoxy, CF₃, keto (=O), cyano and an unsubstituted phenyl radical. If any one of these substituents itself is at least mono-substituted, said substituents may preferably be selected from the group consisting of F, Cl, methyl and methoxy. Preferably the substituted mono- or bicycyclic ringsystem may be substituted with 1, 2, 3, 4 or 5, more preferably with 1, 2 or 3 of the afore mentioned substituents.

If one or more of the residues R¹-R⁴ represents or comprises an aryl radical, which is substituted by one or more substituents, unless defined otherwise, each of these substituents may preferably be selected from the group consisting of hydroxy, halogen, branched or linear C₁₋₄-alkoxy, branched or linear C₁₋₄-alkyl, branched or linear C₁₋₄-perfluoroalkoxy, unsubstituted or at least mono-substituted phenoxy, unsubstituted or at least mono-substituted benzoyl, cyclohexyl, branched or linear C₁₋₄-perfluoroalkyl, NR^{A}R^{B} wherein R^{A}, R^{B} are each independently selected from the group consisting of H, a branched or linear C₁₋₄-alkyl-radical, -CH₂-CH₂-OH and phenyl, carboxy, amido, cyano, -C(H)(OH)(phenyl), -C(H)(OH)(CH₃), nitro, -SO₂NH₂, -CO-C₁₋₄-alkyl, -CO-OC₁₋₄-alkyl, -SO-C₁₋₄-alkyl, -SO₂-C₁₋₄-alkyl, -NH-SO₂-C₁₋₄-alkyl, wherein C₁₋₄-alkyl may be branched or linear, an unsubstituted or at least mono-substituted phenyl or naphthyl radical and unsubstituted or at least mono-substituted furanyl-, thienyl-, pyrrolyl-, imidazolyl-, pyrazolyl-, pyridinyl-, pyrimidinyl-, quinolinyl- and isoquinolinyl radical, more preferably be selected from the group consisting of hydroxy, F, Cl, Br, methyl, ethyl, cyano, -C(H)(OH)(phenyl), -C(H)(OH)(CH₃), methoxy, ethoxy, unsubstituted or at least mono-substituted benzoyl, unsubstituted or at least mono-substituted phenoxy, cyclohexyl, CF₃, -CO-CH₃, -CO-OCH₃, -NR^{A}R^{B} wherein R^{A}, R^{B} are each independently selected from the group consisting of H, a branched or linear C₁₋₄-alkyl-radical, -CH₂-CH₂-OH and phenyl, and an unsubstituted phenyl radical. If any of these substituents itself is at least mono-substituted, said substituents may preferably be selected from the group consisting of F, Cl, methyl and methoxy. Preferably the substituted aryl radical may be substituted with 1, 2, 3, 4 or 5, more preferably with 1, 2 or 3 of the afore mentioned substituents.

If one or more of the residues R¹-R⁴ represents or comprises a heteroaryl radical, which is substituted by one or more substituents, unless defined otherwise, each of these substituents may preferably be selected from the group consisting of hydroxy, halogen, branched or linear C₁₋₄-alkoxy, branched or linear C₁₋₄-alkyl, branched or linear C₁₋₄-perfluoroalkoxy, unsubstituted or at least mono-substituted phenoxy, unsubstituted or at least mono-substituted benzoyl, cyclohexyl, branched or linear C₁₋₄ -perfluoroalkyl, NR^{A}R^{B} wherein R^{A}, R^{B} are each independently selected from the group consisting of H, a branched or linear C₁₋₄-alkyl-radical, -CH₂-CH₂-OH and phenyl, carboxy, amido, cyano, nitro, -C(H)(OH)(phenyl), -C(H)(OH)(CH₃), -SO₂NH₂, -CO-C₁₋₄-alkyl, -CO-OC₁₋₄-alkyl, SO-C₁₋₄-alkyl, SO₂-C₁₋₄-alkyl, -NH-SO₂-C₁₋₄-alkyl, wherein C₁₋₄-alkyl may be branched or linear, an unsubstituted or at least mono-substituted phenyl or naphthyl radical and an unsubstituted or at least mono-substituted furanyl-, thienyl-, pyrrolyl-, imidazolyl-, pyrazolyl-, pyridinyl-, pyrimidinyl-, quinolinyl- and isoquinolinyl radical, more preferably be selected from the group consisting of hydroxy, F, Cl, Br, methyl, ethyl, cyano, methoxy, ethoxy, unsubstituted or at least mono-substituted benzoyl, unsubstituted or at least mono-substituted phenoxy, cyclohexyl, CF₃, -C(H)(OH)(phenyl), -C(H)(OH)(CH₃), -CO-CH₃, -CO-OCH₃, -NR^{A}R^{B} wherein R^{A}, R^{B} are each independently selected from the group consisting of H, a branched or linear C₁₋₄-alkyl-radical, -CH₂-CH₂-OH and phenyl, and an unsubstituted phenyl radical. If any one of these substituents itself is at least mono-substituted, said substituents may preferably be selected from the group consisting of F, Cl, methyl and methoxy. Preferably the substituted heteroaryl radical may be substituted with 1, 2, 3, 4 or 5, more preferably with 1, 2 or 3 of the afore mentioned substituents.

Alkylene groups according to the present invention may preferably be selected from the group consisting of -CH₂-, -CH₂-CH₂-, -CH(CH₃)-, -CH₂-CH₂-CH₂-, -CH(CH₃)-CH₂-CH₂-, -CH₂-CH(CH₃)-CH₂-, -CH₂-CH₂-CH(CH₃)-, -C(CH₃)₂-CH₂-CH₂-, -CH₂-CH₂-C(CH₃)₂- and -CH₂-CH₂-CH₂-CH₂.

If one or more of the residues R¹-R⁵ represents or comprises a cycloaliphatic radical, which contains one or more heteroatoms as ring members, unless defined otherwise, each of these heteroatoms may preferably be selected from the group consisting of N, O, S and P, more preferably from the group consisting of N, O and S. Said cycloaliphatic radical may preferably contain 0, 1, 2 or 3 heteroatoms chosen from the afore mentioned group, more preferably it contains 0 or 1 heteroatoms chosen from the afore mentioned group.

If one or more of the residues R¹-R⁴ represents or comprises an heteroaryl radical, which contains one or more heteroatoms as ring members, unless defined otherwise, each of these heteroatoms may preferably be selected from the group consisting of N, O, S and P, more preferably from the group consisting of N, O and S. Said heteroaryl radical may preferably contain 1, 2 or 3 heteroatoms chosen from the afore mentioned group, preferably it contains 1 or 2 heteroatoms chosen from the afore mentioned group.

Preferred are compounds of general formula (I), wherein R¹, R², R³, R⁴ are each independently selected from the group consisting of H; F; Cl; Br; -CN; -NO₂; -OR⁸; a linear or branched, optionally at least mono-substituted C₁₋₄-alkyl radical, a saturated, optionally at least mono-substituted, optionally at least one heteroatom as ring member containing C₅- or C₆- cycloaliphatic radical, which may be bonded via an optionally at least mono-substituted C₁- or C₂-alkylene group;
more preferably R¹, R², R³, R⁴ are each independently selected from the group consisting of H; F; Cl; Br; -CN; -NO₂; -CH₃; -CH₂CH₃; -CHF₂; -CH₂F; -CF₃; -CF₂CF₃; OR⁸; cyclopentyl and cyclohexyl,
even more preferably R¹, R², R³, R⁴ are each independently selected from the group consisting of H; F; Cl; Br, CH₃ and OR⁸ and R⁵-R⁸, A, a, b and c have the meaning given above, optionally in form of one of their stereoisomers, preferably enantiomers or diastereomers, their racemates or in form of a mixture of at least two of its stereoisomers, preferably enantiomers or diastereomers, in any mixing ratio, or salts, preferably physiologically acceptable salts thereof, or corresponding solvates, respectively.

Also preferred are compounds of general formula (I), wherein R⁵ represents H or a linear or branched C₁₋₆ alkyl radical,
more preferably R⁵ represents H or an alkyl radical selected from the group consisting of methyl, ethyl, n-propyl, iso-propyl, n-butyl, iso-butyl, sec-butyl and tert-butyl and R¹-R⁴, R⁶-R⁸, A, a, b and c have the meaning given above, optionally in form of one of their stereoisomers, preferably enantiomers or diastereomers, their racemates or in form of a mixture of at least two of its stereoisomers, preferably enantiomers or diastereomers, in any mixing ratio, or salts, preferably physiologically acceptable salts thereof, or corresponding solvates, respectively.

Also preferred are compounds of general formula (I), wherein R⁶, R⁷ and R⁸, identical or different, each represent H or a prodrug-moiety selected from the group consisting of
linear or branched C₁₋₃-alkyl,
a P(=O)(OR⁹)₂ group, wherein R⁹ represents a linear or branched C₁₋₄-alkyl radical,
a -(C=O)-O-R¹⁰ group, wherein R¹⁰ represents a linear or branched C₁₋₅-alkyl radical,
a -(C=O)-NH-R¹¹ group, wherein R¹¹ represents a phenyl group, which is mono-substituted with a linear or branched C₁₋₃ alkyl radical,
a -(C=O)-R¹² group, wherein R¹² represents a phenyl group, which is mono-substituted with a -O-(C=O)-C₁₋₃-alkyl radical, an -CH₂-N(C₁₋₄-alkyl)₂ group or a 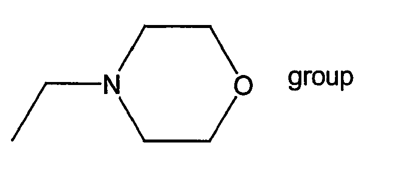 and R¹-R⁵, A, a, b and c have the meaning given above, optionally in form of one of their stereoisomers, preferably enantiomers or diastereomers, their racemates or in form of a mixture of at least two of its stereoisomers, preferably enantiomers or diastereomers, in any mixing ratio, or salts, preferably physiologically acceptable salts thereof, or corresponding solvates, respectively.

Also preferred are compounds of general formula (I), wherein R⁶, R⁷ and R⁸, identical or different, each represent H or a prodrug-moiety selected from the group consisting of
linear or branched C₁₋₃-alkyl,
a P(=O)(OR⁹)₂ group, wherein R⁹ represents methyl or ethyl,
a -(C=O)-O-R¹⁰ group, wherein R¹⁰ represents an alkyl radical selected from the group consisting of methyl, ethyl, n-propyl, iso-propyl, n-butyl, iso-butyl, sec-butyl and tert-butyl,
a -(C=O)-NH-R¹¹ group, wherein R¹¹ represents a phenyl group, which is mono-substituted with methyl or ethyl,
a -(C=O)-R¹² group, wherein R¹² represents a phenyl group, which is mono-substituted with -O-(C=O)-C₁₋₃-alkyl radical in the ortho position or with an -CH₂-N(C₁₋₄-alkyl)₂ in the meta or para position or with a 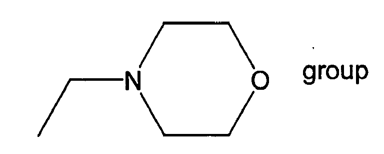 in the meta or para position and R¹-R⁵, A, a, b and c have the meaning given above, optionally in form of one of their stereoisomers, preferably enantiomers or diastereomers, their racemates or in form of a mixture of at least two of its stereoisomers, preferably enantiomers or diastereomers, in any mixing ratio, or salts, preferably physiologically acceptable salts thereof, or corresponding solvates, respectively.

Also preferred are compounds of general formula (I), wherein R⁶, R⁷ and R⁸ each represent hydrogen and R¹-R⁵, A, a, b and c have the meaning given above, optionally in form of one of their stereoisomers, preferably enantiomers or diastereomers, their racemates or in form of a mixture of at least two of its stereoisomers, preferably enantiomers or diastereomers, in any mixing ratio, or salts, preferably physiologically acceptable salts thereof, or corresponding solvates, respectively.

Also preferred are compounds of general formula (I), wherein A represents a -CH₂-group and R¹-R⁴, R⁵, R⁶-R⁸, a, b and c have the meaning given above, optionally in form of one of their stereoisomers, preferably enantiomers or diastereomers, their racemates or in form of a mixture of at least two of its stereoisomers, preferably enantiomers or diastereomers, in any mixing ratio, or salts, preferably physiologically acceptable salts thereof, or corresponding solvates, respectively.

Also preferred are compounds of general formula (I), wherein a represents 1, 2 or 3, more preferably 1 or 2, even more preferably 1 and R¹-R⁴, R⁵, R⁶-R⁸, A, b and c have the meaning given above, optionally in form of one of their stereoisomers, preferably enantiomers or diastereomers, their racemates or in form of a mixture of at least two of its stereoisomers, preferably enantiomers or diastereomers, in any mixing ratio, or salts, preferably physiologically acceptable salts thereof, or corresponding solvates, respectively.

Also preferred are compounds of general formula (I), wherein b represents 0, 1 or 2, more preferably 0 or 1 and R¹-R⁴, R⁵, R⁶-R⁸, A, a and c have the meaning given above, optionally in form of one of their stereoisomers, preferably enantiomers or diastereomers, their racemates or in form of a mixture of at least two of its stereoisomers, preferably enantiomers or diastereomers, in any mixing ratio, or salts, preferably physiologically acceptable salts thereof, or corresponding solvates, respectively.

Also preferred are compounds of general formula (I), wherein c represents 0, 1 or 2, preferably 0 or 1 and R¹-R⁴, R⁵, R⁶-R⁸, A, a and b have the meaning given above, optionally in form of one of their stereoisomers, preferably enantiomers or diastereomers, their racemates or in form of a mixture of at least two of its stereoisomers, preferably enantiomers or diastereomers, in any mixing ratio, or salts, preferably physiologically acceptable salts thereof, or corresponding solvates, respectively.

Also preferred are compounds of general formula (I), wherein at least one of the substituents R¹, R², R³ and R⁴ represents -OR⁸ and the other substituents of R¹, R², R³ and R⁴ and R⁵, R⁶-R⁸, A, a, b and c have the meaning given above, optionally in form of one of their stereoisomers, preferably enantiomers or diastereomers, their racemates or in form of a mixture of at least two of its stereoisomers, preferably enantiomers or diastereomers, in any mixing ratio, or salts, preferably physiologically acceptable salts thereof, or corresponding solvates, respectively.

Also preferred are compounds of general formula (I), wherein one or two of the substituents R¹, R², R³ and R⁴ represent -OR⁸ and the other substituents of R¹, R², R³ and R⁴ and R⁵, R⁶-R⁸, A, a and b have the meaning given above, optionally in form of one of their stereoisomers, preferably enantiomers or diastereomers, their racemates or in form of a mixture of at least two of its stereoisomers, preferably enantiomers or diastereomers, in any mixing ratio, or salts, preferably physiologically acceptable salts thereof, or corresponding solvates, respectively.

Also preferred are compounds of general formula (I), wherein one or two of the substituents R¹, R², R³ and R⁴ represent -OR⁸ and b and c each represent 0, more peferably one of the substituents R¹, R², R³ and R⁴ represents -OR⁸ and b and c each represent 0 and in each case the other substituents of R¹, R², R³ and R⁴ and R⁵, R⁶-R⁸, A and a have the meaning given above, optionally in form of one of their stereoisomers, preferably enantiomers or diastereomers, their racemates or in form of a mixture of at least two of its stereoisomers, preferably enantiomers or diastereomers, in any mixing ratio, or salts, preferably physiologically acceptable salts thereof, or corresponding solvates, respectively.

Most preferred are 1,4-disubstituted piperidine compounds of general formula (I) selected from the group consisting of:
[1] 2-[4-(3-Hydroxy-2-hydroxymethyl-phenylamino)-piperidin-1-yl]-N-(9-methyl-9H-carbazol-3-yl)-acetamide,
[2] 2-[4-(4-Hydroxy-2-hydroxymethyl-phenylamino)-piperidin-1-yl]-N-(9-methyl-9H-carbazol-3-yl)-acetamide,
[3] 2-[4-(5-Hydroxy-2-hydroxymethyl-phenylamino)-piperidin-1-yl]-N-(9-methyl-9H-carbazol-3-yl)-acetamide,
[4] 2-[4-(6-Hydroxy-2-hydroxymethyl-phenylamino)-piperidin-1-yl]-N-(9-methyl-9H-carbazol-3-yl)-acetamide,
[5] 2-[4-(3-Hydroxy-2-hydroxymethyl-phenylamino)-piperidin-1-yl]-N-(9-ethyl-9H-carbazol-3-yl)-acetamide,
[6] 2-[4-(4-Hydroxy-2-hydroxymethyl-phenylamino)-piperidin-1-yl]-N-(9-ethyl-9H-carbazol-3-yl)-acetamide,
[7] 2-[4-(5-Hydroxy-2-hydroxymethyl-phenylamino)-piperidin-1-yl]-N-(9-ethyl-9H-carbazol-3-yl)-acetamide and
[8] 2-[4-(6-Hydroxy-2-hydroxymethyl-phenylamino)-piperidin-1-yl]-N-(9-ethyl-9H-carbazol-3-yl)-acetamide,
optionally in form of a salt, preferably a physiologically acceptable salt, more preferably in form of a physiologically acceptable acid addition salt, most preferably a hydrochloride salt, or a corresponding solvate.

In a further aspect the present invention also provides a process for the preparation of 1,4-disubstituted piperidine compounds of general formula (I), wherein at least one compound of general formula (II), 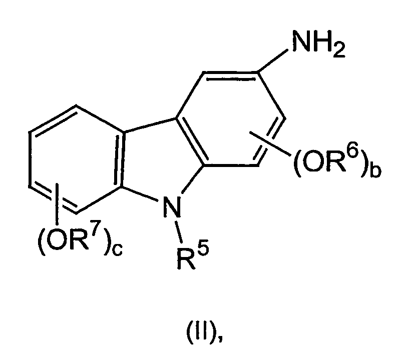 wherein R⁵, R⁶ and R⁷, b and c have the meaning given above; is reacted with at least one compound of general formula (III), 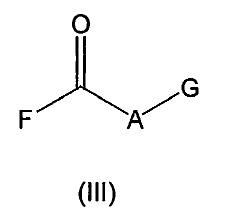 wherein A has the meaning according given above, F represents halogen, preferably chlorine, hydroxy or an O-acyl group and G represents halogen, preferably chlorine, in a suitable reaction medium and preferably in the presence of at least one base and/or at least one auxiliary agent, and reacting the so obtained compound of general (IV) 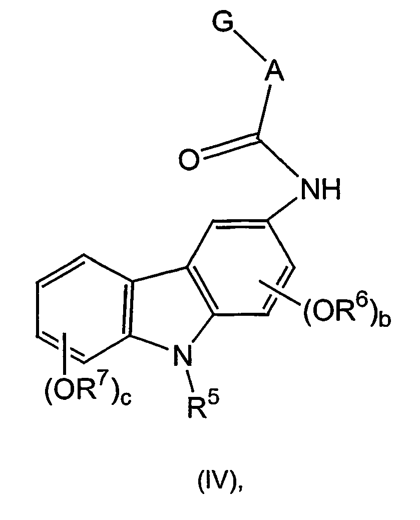 wherein A, G, R⁵, R⁶ and R⁷, b and c have the above defined meaning, with at least one piperidine compound of general formula (V) and/or a salt, preferably a hydrochloride salt thereof, 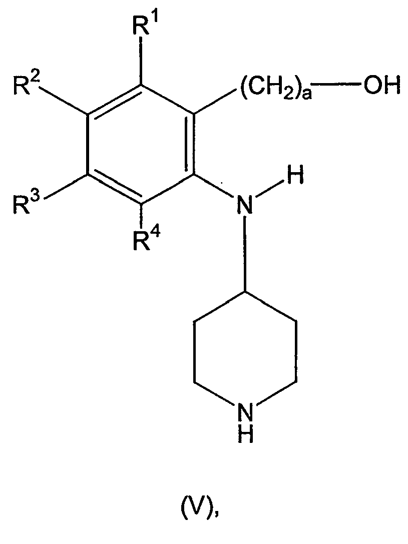 wherein R¹ to R⁴ and a have the meaning given above, in a suitable reaction medium, optionally in the presence of at least one base and/or at least one auxiliary agent, to yield a compound of general formula (I), wherein R¹-R⁷, A, a, b and c have the meaning as given above.

According to the invention, the process may be illustrated as an example by the following reaction scheme 1: 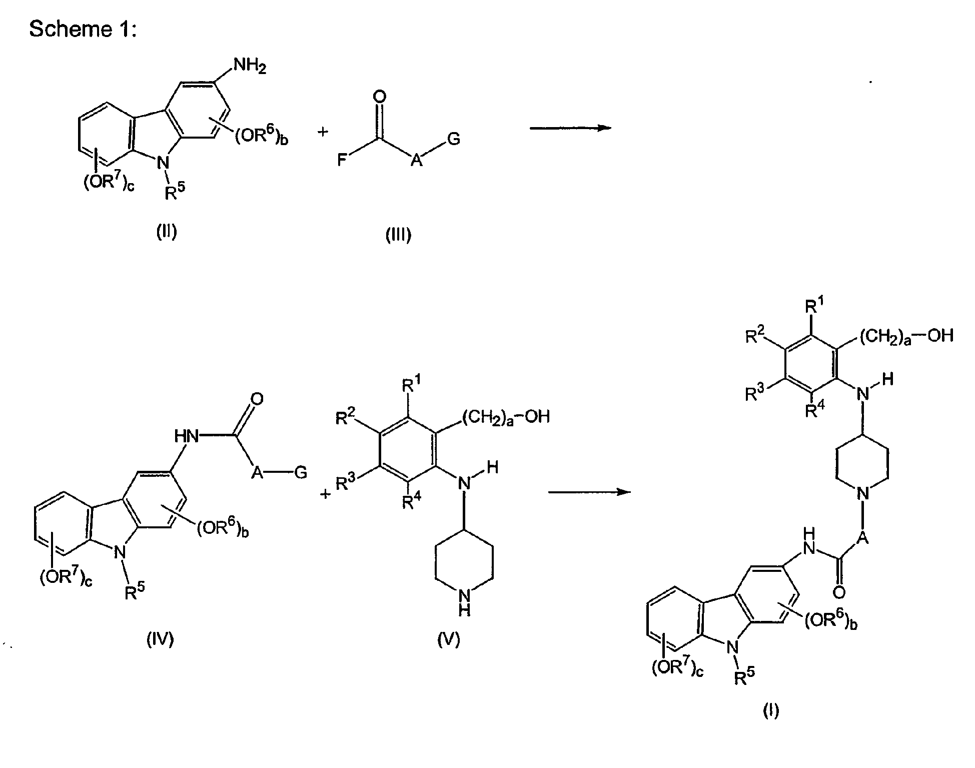 wherein R¹-R⁷, A, a, b and c have the meaning as given above.

Suitable reaction media are e.g. organic solvents, such as ethers, preferably diethyl ether, dioxane, tetrahydrofurane, dimethyl glycol ether, or alcohols, e.g. methanol, ethanol, propanol, isopropanol, butanol, isobutanol, tert-butanol, or hydrocarbons, preferably benzene, toluene, xylene, hexane, cyclohexane, petroleum ether, or halogenated hydrocarbons, e.g. dichloromethane, trichloromethane, tetrachloromethane, dichloroethylene, trichloroethylene, chlorobenzene or/and other solvents, preferably ethyl acetate, triethylamine, pyridine, dimethylsulfoxide, dimethylformamide, hexamethylphosphoramide, acetonitril, acetone or nitromethane, are included. Mixtures based one or more of the aforementioned solvents may also be used.

Bases that may be used in the processes according to the present invention are generally organic or inorganic bases, preferably alkali metal hydroxides, e.g. sodium hydroxide or potassium hydroxide, or obtained from other metals such as barium hydroxide or different carbonates, preferably potassium carbonate, sodium carbonate, calcium carbonate, or alkoxides, e.g. sodium methoxide, potassium methoxide, sodium ethoxide, potassium methoxide, potassium ethoxide or potassium tert-butoxide, or organic amines, preferably triethylamine, diisopropyethylamine or heterocycles, e.g. 1,4-diazabicyclo[2.2.2] octane, 1,8-diazabicyclo[5.4.0]undec-7-ene pyridine, diamino pyridine, dimethylaminopyridine, methylpiperidine or morpholine. Alkali metals and their hydrides such as sodium or its hydrides, e.g. sodium hydride, may also be used. Mixtures based one or more of the aforementioned bases may also be used.

The above mentioned bases may be used for the process as auxiliary agents, when appropriate. Other suitable auxiliary agents for the above mentioned reactions are, for example, dehydrating agents like carbodiimides, e.g. diisopropylcarbodiimide, cyclohexylcarbodiimide or N-(3-dimethylaminopropyl)-N'-ethylcarbodiimide hydrochloride, or carbonylic compounds, e.g. carbonyldiimidazol or compounds like isobutylchloroformiate or methansulfonyl chloride, among others. These reagents are generally used in amounts from 0.5 to 5 mol versus 1 mol of the corresponding reactands. These bases are generally used in amounts from 0.05 to 10 mol versus 1 mol of the corresponding reactands.

During some of the synthetic reactions described or while preparing the compounds of general formulas (I), (II), (III), (IV), and (V) the protection of sensitive groups or of reagents may be necessary and/or desirable. This can be performed by using conventional protective groups like those described in the literature, e.g. in Protective groups in Organic Chemistry, ed. J.F.W. McOmie, Plenum Press, 1973; T.W. Greene & P.G.M. Wuts, Protective Groups in Organic Chemistry, John Wiley & Sons, 1991. The protective groups may also be eliminated as convenient by means well-known to those skilled in the art. The respective parts of the literature description are hereby incorporated by reference and form part of the present disclosure.

The compounds of general formulas (II), (III), (IV) and (V) are either commercially available or can be produced according to methods known to those skilled in the art. The reaction of compounds of general formulas (IV) and (V) to yield 1,4-disubstituted piperidine compounds of general formula (I) may also be facilitated by conventional methods known to those skilled in the art.

The compounds of general formula (IV) are commercially available or may be produced by conventional methods known to those skilled in the art. In particular, the respective compound of general formula (II) may be reacted with chloroacetyl chloride or the respective compound of general formula (III) in the presence of an organic reaction medium, preferably dichloromethane and a base, preferably triethylamine and/or diisopropylethylamine as depicted in scheme 2.

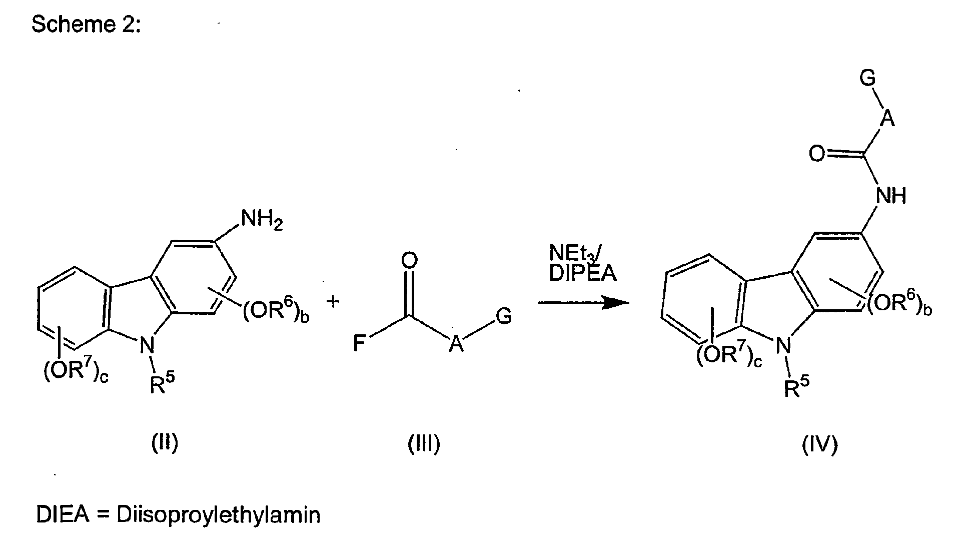

The preparation of compounds of general formula (Va), wherein R¹-R⁴ have the meaning as given above and their use for the preparation of compounds of general formula (I) is illustrated in scheme 3 given below:

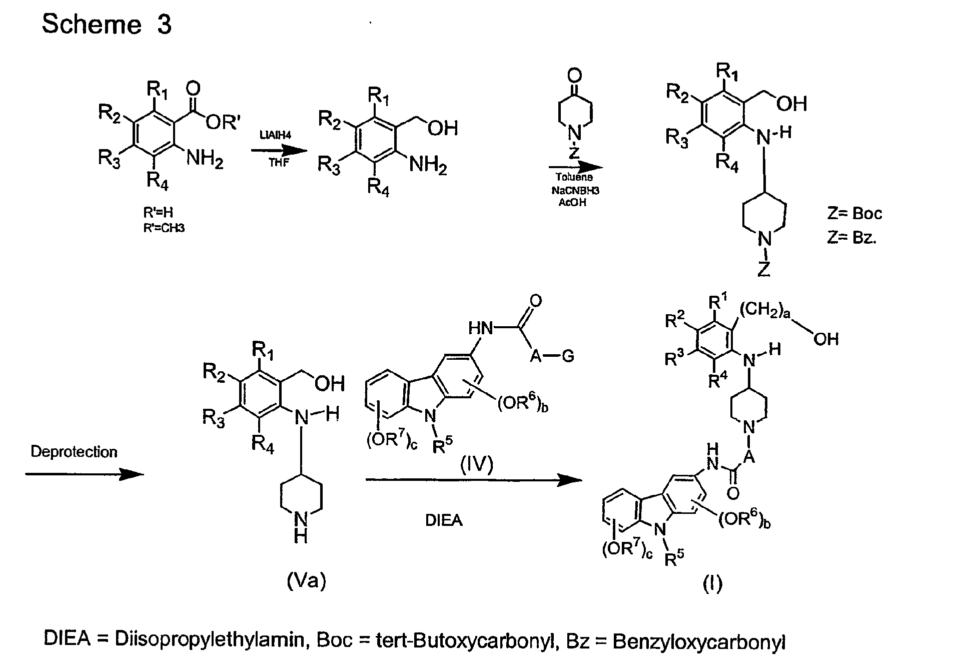

In a further aspect the present invention also provides a process for the preparation of salts of 1,4-disubstituted piperidine compounds of general formula (I), wherein at least one compound of general formula (I) is reacted with an inorganic and/or organic acid, preferably in the presence of a suitable reaction medium. Suitable reaction media are the ones given above. Suitable inorganic acids are for example hydrochloric acid, hydrobromic acid, phosphoric acid, sulfuric acid, nitric acid, suitable organic acids are e.g. citric acid, maleic acid, fumaric acid, tartaric acid, or derivatives thereof, such as p-toluenesulfonic acid, methanesulfonic acid or camphersulfonic acid.

In yet a further aspect the present invention also provides a process for the preparation of salts of 1,4-disubstituted piperidine compounds of general formula (I), wherein at least one compound of general formula (I) having at least one acidic group is reacted with one or more suitable bases, preferably in the presence of a suitable reaction medium. Suitable bases are e.g. hydroxides, carbonates or alkoxides, which include suitable cations, derived e.g. from alkaline metals, alkaline earth metals or organic cations, e.g. [NHₙR₄₋ₙ]⁺, wherein n is 0, 1, 2, 3 or 4 and R represents a branched or unbranched C₁₋₄-alkyl-radical.

Solvates, preferably hydrates, of the 1,4-disubstituted piperidine compounds of general formula (I), or corresponding stereoisomers, or corresponding salts may also be obtained by standard procedures known to those skilled in the art.

If the 1,4-disubstituted piperidine compounds of general formula (I) are obtained in form of a mixture of stereoisomers, particularly enantiomers or diastereomers, said mixtures may be separated by standard procedures known to those skilled in the art, e.g. chromatographic methods or crystallization with chiral reagents.

The purification and isolation of the 1,4-disubstituted piperidine compounds of general formula (I) or a corresponding stereoisomer, or a corresponding salt, or corresponding solvate respectively, if required, may be carried out by conventional methods known to those skilled in the art, e.g. chromatographic methods or recrystallization.

The 1,4-disubstituted piperidine compounds of general formula (I), their stereoisomers or the respective salts or solvates are toxicologically acceptable and are therefore suitable as pharmaceutical active substances for the preparation of medicaments.

Surprisingly, it has been found that the 1,4-disubstituted piperidine compounds of general formula (I) have affinity for neuropeptide Y receptors, in particular for neuropeptide Y 5 (NPY5) receptors. Moreover, the compounds according to the present invention have surprisingly been found to show significant appetite suppressing effects in rats, if administered orally or parenterally. It is particularly suprising that the compounds of general formula (I) are pharmacologically active, if administered orally.

The present invention therefore also provides for a medicament comprising at least one 1,4-disubstituted piperidine compound of general formula (I), optionally in form of one of its stereoisomers, preferably enantiomers or diastereomers, its racemate or in form of a mixture of at least two of its stereoisomers, preferably enantiomers or diastereomers, in any mixing ratio, or a physiologically acceptable salt thereof, or a solvate, respectively, and optionally one or more pharmaceutically acceptable adjuvants.

Furthermore, the present invention also provides for a pharmaceutical composition comprising at least one 1,4-disubstituted piperidine compound of general formula (I), optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, and optionally one or more pharmaceutically acceptable adjuvants, which is not yet formulated into a medicament.

Preferably the medicament is suitable for the regulation of neuropeptide Y receptors, preferably of neuropeptide Y 5 (NPY5) receptor, for the regulation of appetite, for the regulation of body weight, for the prophylaxis and/or treatment of disorders related to food ingestion, preferably selected from the group consisting of obesity, anorexia, cachexia, bulimia, diabetes (particularly type (II) diabetes), for the improvement of cognition (cognitive enhancement); for the prophylaxis and/or treatment of disorders of the peripheral nervous system; for the prophylaxis and/or treatment of disorders of the central nervous system; for the prophylaxis and/or treatment of arthritis; for the prophylaxis and/or treatment of epilepsy; for the prophylaxis and/or treatment of anxiety; for the prophylaxis and/or treatment of depression; for the prophylaxis and/or treatment of cognitive disorders, more preferably memory disorders; for the prophylaxis and/or treatment of cardiovascular diseases; for the prophylaxis and/or treatment of pain; for the prophylaxis and/or treatment of hypertensive syndrom; for the prophylaxis and/or treatment of inflammatory diseases; for the prophylaxis and/or treatment of immune diseases; for the prophylaxis and/or treatment of panic attacks; and for the prophylaxis and/or treatment of bipolar disorders.

The present invention also provides for the use of at least one 1,4-disubstituted piperidine compound of general formula (I), optionally in form of one of its stereoisomers, preferably enantiomers or diastereomers, its racemate or in form of a mixture of at least two of its stereoisomers, preferably enantiomers or diastereomers, in any mixing ratio, or a physiologically acceptable salt thereof, or a solvate, respectively, for the manufacture of a medicament for the regulation of neuropeptide Y receptors, preferably of neuropeptide Y 5 (NPY5) receptor, for the regulation of appetite, for the regulation of body weight, for the prophylaxis and/or treatment of disorders related to food ingestion, preferably selected from the group consisting of obesity, anorexia, cachexia, bulimia, diabetes (particularly type (II) diabetes), for the improvement of cognition (cognitive enhancement); for the prophylaxis and/or treatment of disorders of the peripheral nervous system; for the prophylaxis and/or treatment of disorders of the central nervous system; for the prophylaxis and/or treatment of arthritis; for the prophylaxis and/or treatment of epilepsy; for the prophylaxis and/or treatment of anxiety; for the prophylaxis and/or treatment of depression; for the prophylaxis and/or treatment of cognitive disorders, preferably memory disorders; for the prophylaxis and/or treatment of cardiovascular diseases; for the prophylaxis and/or treatment of pain; for the prophylaxis and/or treatment of hypertensive syndrom; for the prophylaxis and/or treatment of inflammatory diseases; for the prophylaxis and/or treatment of immune diseases; for the prophylaxis and/or treatment of panic attacks; and for the prophylaxis and/or treatment of bipolar disorders.

The medicament according to the present invention is particularly suitable for the administration to mammals, including humans. The medicament can be administered to patients of all ages, namely children, adolescents and adults. The composition of the medicament may vary depending on the route of administration.

The preparation of the corresponding pharmaceutical compositions as well as the formulated medicaments can be carried out by means of conventional methods known in the prior art, for example, from the indices of "Pharmaceutics: The Science of Dosage Forms", Second Edition, Aulton, M.E. (ED. Churchill Livingstone, Edinburgh (2002)); "Encyclopedia of Pharmaceutical Technology", Second Edition, Swarbrick, J. and Boylan, J.C. (Eds.), Marcel Dekker, Inc. New York (2002); "Modern Pharmaceutics", Fourth Edition, Banker G.S. and Rhodes C.T. (Eds.) Marcel Dekker, Inc. New York (2002), and "The Theory and Practice of Industrial Pharmacy", Lachman L., Lieberman H. and Kanig J. (Eds.), Lea & Febiger, Philadelphia (1986). The respective literature descriptions are incorporated as a reference and are part of this disclosure.

The pharmaceutical compositions, as well as the formulated medicaments prepared according to the present invention, can, in addition to at least one compound of general formula (I), optionally in the form of one of its stereoisomers, preferably enantiomers or diastereomers, its racemate, or in form of a mixture of at least two of its stereoisomers, preferably enantiomers or diastereomers, in any mixing ratio, or a corresponding physiologically acceptable salt thereof or a corresponding solvate thereof, comprise other conventional auxiliary substances known in the prior art, preferably excipients, fillers, solvents, diluents, dyes, coating agents, matrix forming agents and/or binders.

As the skilled persons in the art also knows, the choice of the auxiliary substances and the amounts thereof depend on the intended administration route, for example, rectal, intravenous, intraperitoneal, intramuscular, intranasal, oral, buccal or topical.

Medicaments suitable for oral administration are, for example, tablets, coated tablets, capsules or multiparticulates, preferably granules or pellets, optionally compressed into tablets, filled in capsules or suspended in suitable liquids.

Medicaments suitable for parenteral, topical or inhalatory administration may preferably be chosen from the group consisting of solutions, suspensions, quickly reconstitutable dry preparations and also sprays.

Medicaments suitable for oral or percutaneous use can release the compounds of general formula (I) in a sustained manner, the preparation of these sustained release medicaments generally being known in the prior art.

Suitable sustained release forms, as well as the materials and methods for the preparation thereof, are known in the prior art, for example from the indices of "Modified-Release Drug Delivery Technology", Rathbone, J.JI, Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York (2000); "Controlled Drug Delivery", Vol. I, Basic Concepts, Bruck, S.D. (Ed.), CRD Press, Inc., Boca Raton (1983), and by Takada, K. and Yoshikawa, H., "Oral Drug Delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encyclopedia of Controlled Drug Delivery, Mathiowitz, E. (Ed.), John Wiley & Sons, Inc., New York (1999), Vol. 2, 698-728. The respective literature references are incorporated by reference and form part of the disclosure.

The medicament of the present invention may also have at least one enteric coating, which dissolves according to the pH. As a result of this coating, the medicament may pass through the stomach without dissolving, and the compounds of general formula I are only released in the intestinal tract. The enteric coating preferably dissolves at a pH of between 5 and 7.5. The materials and methods suitable for preparing enteric coatings are also known in the prior art.

The above mentioned compositions include preferably 1 to 60 % by weight of one or more of the 1,4-disubstituted piperidine compound of general formula (I), optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, and 40 to 99 % by weight of the appropriate pharmaceutical vehicle(s).

The daily dosage for humans and animals may vary depending on factors that have their basis in the respective species or other factors, such as age, weight or degree of illness and so forth. The daily dosage for mammals including humans usally ranges from 1 milligram to 5000 milligram, preferably 1 to 3000 mg, more preferably 1 to 2000 mg of substance to be administered during one or several intakes.

### Pharmacological Methods:

### Neuropeptide Y₅ Receptor binding studies:

The methods used for membrane preparation and binding are similar to those described by Y. Hu, B. T. Bloomquist et al. in Y. Hu, B. T. Bloomquist et al., The Journal of Biological Chemistry, 1996, 271, 26315-26319 with modifications. Said literature description is herewith incorporated by reference and forms part of the disclosure. Cells C6 were transfected with the rat Y5 receptor. The cells were grown under standard culture conditions in 150 cm² dishes and they were harvested using a rubber scraper and 10 ml PBS. The cells from five dishes were collected and centrifuged 2.500 g for 5 min (4°C). The pellet was washed by resuspending in 3 ml buffer (Tris-HCl 10 mM, pH 7.4), homogenized using a Potter S homogenizer, 10 strokes at 600 rpm and centrifuged 48.000 g for 20 min (4°C). The pellet was resuspended in 8 ml membrane buffer (Tris-HCl 25 mM, NaCl 120 mM, KCl 5 mM, KH₂PO₄ 1,2 mM, CaCl₂ 2,5 mM, MgSO₄ 1,2 mM, BSA 0,15 mg/ml, Bacitracine 0,5 mg/ml, pH 7,4) and rehomogenized using the Potter S, 10 strokes at 600 rpm. The protein concentration in the incubation was 40 µg/ml. The radioligand was [¹²⁵I]-PYY (100 pM) in a total incubation volume of 200 µl. Following incubation at 25°C for 2 h, the reaction was stopped by addition of 5 ml ice-cold buffer (Tris-HCl 25 mM, NaCl 120 mM, KCI 5 mM, KH₂PO₄ 1,2 mM, CaCl₂ 2,5 mM, MgSO₄ 1,2 mM, pH 7,4) and rapid filtration in a Harvester Brandell Cell using filters (Schleicher & Schuell GF 3362) pretreated for two hours with 0,5% polyethyleneimine. Filters were washed one time with 5 ml ice-cold buffer. The filters were placed into plastic scintilation vials and 5 ml scintilation cocktail Ecoscint H were added. The quantity of radioactivity present was determined in a Wallac Winspectral 1414 counter. Non specific binding was determined in the presence of 1 µM de pNPY. All binding assays were done in triplicate.

### Behavioural model (Food intake measurements)

In this test the effect of the compounds of general formula (I) on food and water intake in male rats can be determined.

### Animals:

128 male Sprague Dawley Rats (aged 6 weeks, approximately 190 g; obtained from Charles River, Germany) were used. The rats arrived 32 at a time. Upon arrival they were be housed 3 per cage for one week and subsequently transferred to individual cages mounted with feeders containing powdered chow. During the single housing period, rats were handled daily to accustom them to the injection procedure. From the arrival date, rats are kept under a 12/12 UD cycle lights on at 0300 and in temperature and humidity controlled rooms.

### Treatment groups and randomization

Two weeks after the arrival, the rats were transferred to MANI Feedwin cages and randomized into 4 weight-matched groups, that is 8 rats per group. Rats had ad libitum access to a powdered diet (Atromin rodent chow, C.Petersen Ringsted) and tap water. In addition, body weight was monitored daily.

Rats were subjected to a maximum of 4 injections, each separated by at least 3 days. If carry over effects were still present at that time injections were postponed further. All compounds were administered in three doses: 5, 30 and 60 mg/kg. All compounds were administered p.o. by gavage (gavage volume of 5-8 ml/kg, determined by the solubility of the compound).
Group 1 Vehicle
Group 2 testing compound (I) 5 mg/kg
Group 3 testing compound (I) 30 mg/kg
Group 4 testing compound (I) 60 mg/kg

### Experimental procedure

For 2 days prior to transfer to the MANI Feedwin cages, in addition to the daily handling procedure rats were gavaged daily with vehicle. Baseline food intake (digital balance) and lick counts were monitored from day 1 to day 3. First day of injection was day 3. Prior to lights out (14.30 PM) the rats were administered the testing compound and vehicle by gavage. Food intake (digital balance) and water intake (registered as lick counts) were monitored online every 5^{th} minute for 48 hours following the time of injection or until the effect of the drug had worn off.

Testing compounds (I) with significant appetite suppressing effects, i. e. effective compounds, at any of the following time points 1, 4, 6, 12, 18, 24, 48 h after the injection were re-administered to the same group of rats in a randomized manner so that no rat received the same dosage twice.

In addition, the following analysis was carried out for effective testing compounds:
- Locomotor activity (consecutive beam streaks) analyzed for 48 hours following administration of the testing compounds at the same time as food intake was registered
- Meal microstructure analysis based on the food and licking data from the experiment in 5 minutes intervals. The meal size, the meal duration, the interval between two meals and the meal numbers was analyzed during the first 24 hours after administration.

The following examples are given to illustrate the present invention, but they do not limit the scope of the present invention.

### Examples:

### EXAMPLE A :

### 2-Chloro-N-(9-methyl-9H-carbazol-3-yl)-acetamide

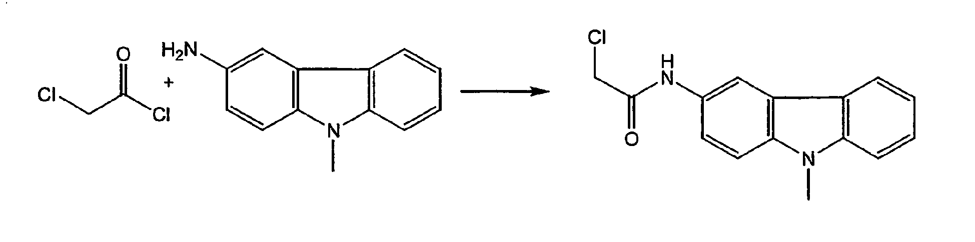

A solution of 3-amino-9-methyl-9H-carbazol (10 mmols), triethylamine (2.07 ml, 15 mmols), in 25 ml of dried dichloromethane, is cooled to 10° C and a solution of chloroacetyl chloride (10.5 mmoles) in 10 ml of dried dichloromethane is then added drop by drop. The resulting mixture is kept stirring for 1 hour at room temperature overnight.The mixture is washed with 2x30 ml of water, dried over sodium sulfate and evaporated to give 2,5 g of 2-Chloro-N-(9-methyl-9H-carbazol-3-yl)-acetamide.

### EXAMPLE 1 :

### 2-[4-(3-Hydroxy-2-hydroxymethyl-phenylamino)-piperidin-1-yl]-N-(9-methyl-9H-carbazol-3-yl)-acetamide

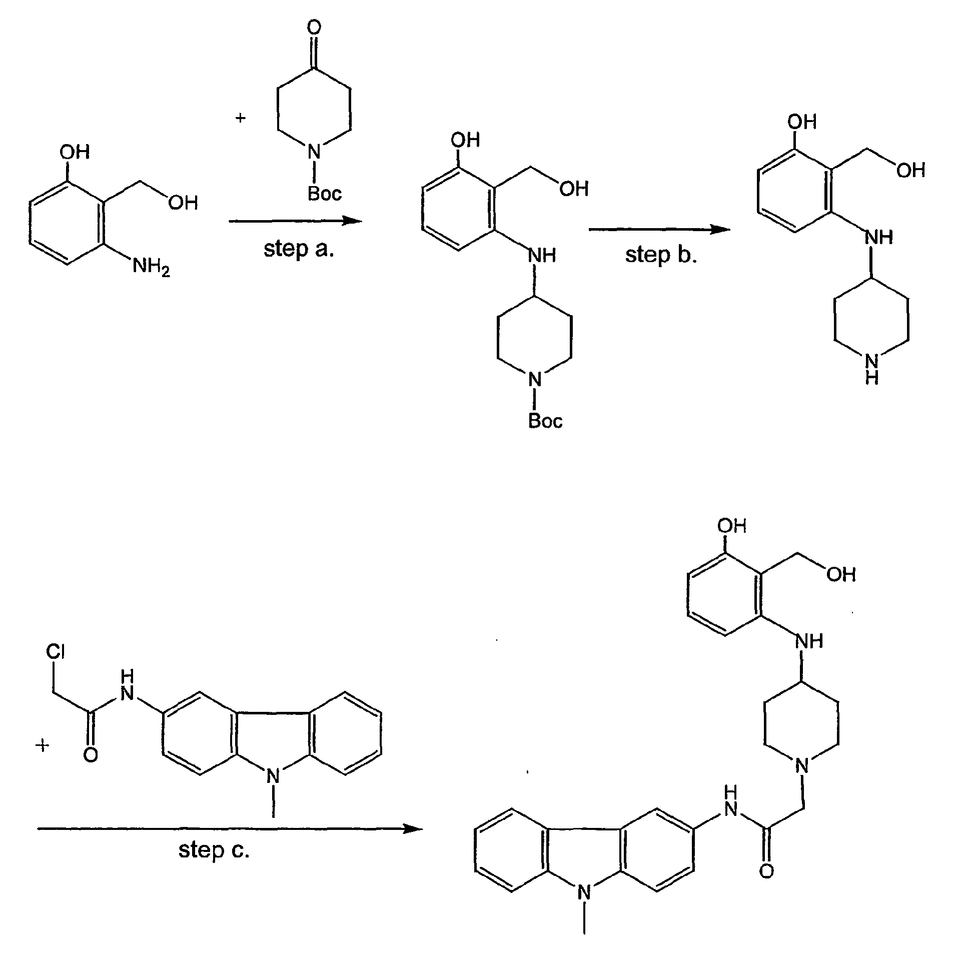

### step a)

A solution of 1-(*tert*-butyloxycarbonyl)-4-piperidinone (0.01 mol), 3-Amino-2-hydroxymethyl-phenol (0.011 mol) and acetic acid (1.4 ml, 0.022 mol) in dried toluene (50 mL) were heated to reflux, removing the water by means of azeotropic distillation with a Dean-Stark, over 30 hours. Then, the mixture was cooled and concentrated under vacuum to the half of the volume. NaBH₃CN (2 g, 0.032 mol) and dried THF (30 mL) is added to a resulting solution.

Afterwards, acetic acid (1 mL, 0.017 mol) was added slowly and the reaction mixture was stirred at room temperature over 24 hours. The mixture was concentrated under vacuum and the residue was dissolved in ethyl acetate (75 mL), washed with a saturated NaHCO₃ (4 x 25 mL) and a saturated NaCl solution (25 mL), dried and evaporated to dryness. This raw material was used in the following step.

### step b)

A solution of 3.2 g of the raw material obtained in the previous step a) in 40 mL of dried ethyl acetate, was cooled to 0°C. Then a 5 M hydrogen chloride solution in ethyl ether (40 mL) was added and the resulting mixture was kept at 0°C over 4 hours. The solvent was evaporated and the residue was suspended in water and was alcalinized with sodium hydroxide, and was extracted with chloroform (3 x 20 mL), the combined organic extracts were washed with water, dried over sodium sulfate and evaporated. The raw material was purified via column cromatography by eluting with chloroform:methanol 9:1 (vol/vol). In this way 1,3 g of a yellow solid were obtained.

### step c)

A mixture of 3-N-(4-Amino-piperidin)-2-hydroxymethyl-phenol (4.70 mmol), 2-Chloro-N-(9-methyl-9H-carbazol-3-yl)-acetamide (5 mmol) and K₂CO₃ (1380 mg, 10 mmol) in DMF (40 mL) was stirred at 10°C for 2 hours and then at room temperature overnight. The reaction mixture was added to 50 mL water and 100 mL ethyl acetate, the organic phase was decanted and washed with water (3 x 50 mL), dried over sodium sulfate and a 2.8 M hydrogen chloride solution in absolute ethanol (1.80 mL) was added, to precipitate the hydrochloride, which was filtered off and washed with ethyl acetate to obtain the compound 2-[4-(3-Hydroxy-2-hydroxymethylphenylamino)-piperidin-1-yl]-N-(9-methyl-9H-carbazol-3-yl)-acetamide with a yield of 70%.

The compounds according to the following examples 2-8 have been prepared as described above for the compound according to example 1.

### Example 2:

### 2-[4-(4-Hydroxy-2-hydroxymethyl-phenylamino)-piperidin-1-yl]-N-(9-methyl-9H-carbazol-3-yl)-acetamide

### Example 3:

### 2-[4-(5-Hydroxy-2-hydroxymethyl-phenylamino)-piperidin-1-yl]-N-(9-methyl-9H-carbazol-3-yl)-acetamide

### Example 4:

### 2-[4-(6-Hydroxy-2-hydroxymethyl-phenylamino)-piperidin-1-yl]-N-(9-methyl-9H-carbazol-3-yl)-acetamide

### Example 5:

### 2-[4-(3-Hydroxy-2-hydroxymethyl-phenylamino)-piperidin-1-yl]-N-(9-ethyl-9H-carbazol-3-yl)-acetamide

### Example 6:

### 2-[4-(4-Hydroxy-2-hydroxymethyl-phenylamino)-piperidin-1-yl]-N-(9-ethyl-9H-carbazol-3-yl)-acetamide

### Example 7:

### 2-[4-(5-Hydroxy-2-hydroxymethyl-phenylamino)-piperidin-1-yl]-N-(9-ethyl-9H-carbazol-3-yl)-acetamide

### Example 8:

2-[4-(6-Hydroxy-2-hydroxymethyl-phenylamino)-piperidin-1-yl]-N-(9-ethyl-9H-carbazol-3-yl)-acetamide.

## Claims

1. 1,4-disubstituted piperidine compounds of general formula (I), 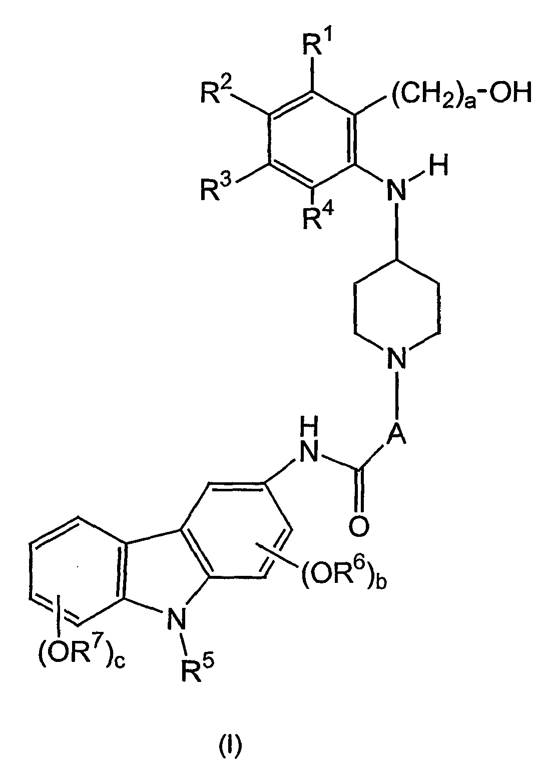 wherein
a represents 0, 1, 2, 3 or 4,
b represents 0, 1, 2 or 3,
c represents 0, 1, 2, 3 or 4,
R¹, R², R³, R⁴ are each independently selected from the group consisting of hydrogen; halogen; -CN; -NO₂; -OR⁸; a linear or branched, saturated or unsaturated, optionally at least mono-substituted aliphatic radical; a saturated or unsaturated, optionally at least mono-substituted, optionally at least one heteroatom as ring member containing cycloaliphatic radical, which may be bonded via an alkylene group; or an optionally at least mono-substituted aryl- or heteroaryl radical, which may be bonded via an alkylene group and/or which may be condensed with an optionally at least mono-substituted, saturated or unsaturated mono- or bicyclic ring system,
R⁵ represents hydrogen, a linear or branched, saturated or unsaturated, optionally at least mono-substituted aliphatic radical, or a saturated or unsaturated, optionally at least mono-substituted cycloaliphatic radical,
R⁶, R⁷ and R⁸, identical or different, each represent hydrogen or a prodrug-moiety,
A represents a -CH₂- or -CH₂-CH₂- group,
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

2. Compounds according to claim 1, **characterized in that** R¹, R², R³, R⁴ are each independently selected from the group consisting of H; F; Cl; Br; -CN; -NO₂; -OR⁸; a linear or branched, saturated or unsaturated, optionally at least mono-substituted C₁₋₆-aliphatic radical; a saturated or unsaturated, optionally at least mono-substituted, optionally at least one heteroatom as ring member containing C₃₋₈-cycloaliphatic radical, which may be bonded via a C₁₋₃-alkylene group; or an optionally at least mono-substituted aryl- or heteroaryl radical, which may be bonded via a C₁₋₃-alkylene group and/or which may be condensed with an optionally at least mono-substituted, saturated or unsaturated mono- or bicyclic ring system,
R⁵ represents hydrogen, a linear or branched, saturated or unsaturated, optionally at least mono-substituted C₁₋₆-aliphatic radical, or a saturated or unsaturated, optionally at least mono-substituted C₃₋₈-cycloaliphatic radical,
R⁶, R⁷ and R⁸, identical or different, each represent hydrogen or a prodrug-moiety,
A represents a -CH₂- or-CH₂-CH₂- group.

3. Compounds according to claim 1 or 2, **characterized in that** R¹, R², R³, R⁴ are each independently selected from the group consisting of H; F; Cl; Br; -CN; -NO₂; -OR⁸; a linear or branched, optionally at least mono-substituted C₁₋₄-alkyl radical, a saturated, optionally at least mono-substituted, optionally at least one heteroatom as ring member containing C₅- or C₆- cycloaliphatic radical, which may be bonded via an optionally at least mono-substituted C₁- or C₂-alkylene group;
preferably R¹, R², R³, R⁴ are each independently selected from the group consisting of H; F; Cl; Br; -CN; -NO₂; -CH₃; -CH₂CH₃; -CHF₂; -CH₂F; -CF₃; -CF₂CF₃; OR⁸; cyclopentyl and cyclohexyl,
more preferably R¹, R², R³, R⁴ are each independently selected from the group consisting of H; F; Cl; Br, CH₃ and OR⁸.

4. Compounds according to any one of claims 1 to 3, **characterized in that** R⁵ represents H or a linear or branched C₁₋₆ alkyl radical,
preferably R⁵ represents H or an alkyl radical selected from the group consisting of methyl, ethyl, n-propyl, iso-propyl, n-butyl, iso-butyl, sec-butyl and tert-butyl.

5. Compounds according to any one of claims 1 to 4 **characterized in that** R⁶, R⁷ and R⁸, identical or different, each represent H or a prodrug-moiety selected from the group consisting of
linear or branched C₁₋₃-alkyl,
a P(=O)(OR⁹)₂ group, wherein R⁹ represents a linear or branched C₁₋₄-alkyl radical,
a -(C=O)-O-R¹⁰ group, wherein R¹⁰ represents a linear or branched C₁₋₅-alkyl radical,
a -(C=O)-NH-R¹¹ group, wherein R¹¹ represents a phenyl group, which is mono-substituted with a linear or branched C₁₋₃ alkyl radical,
a -(C=O)-R¹² group, wherein R¹² represents a phenyl group, which is mono-substituted with a -O-(C=O)-C₁₋₃-alkyl radical, an -CH₂-N(C₁₋₄-alkyl)₂ group or a

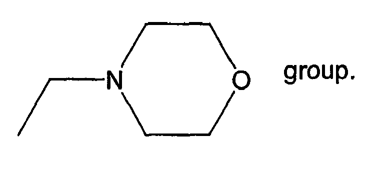

6. Compounds according to claim 5, **characterized in that** R⁶, R⁷ and R⁸, identical or different, each represent H or a prodrug-moiety selected from the group consisting of
linear or branched alkyl selected from the group consisting of methyl, ethyl, n-propyl and iso-propyl,
a P(=O)(OR⁹)₂ group, wherein R⁹ represents methyl or ethyl,
a -(C=O)-O-R¹⁰ group, wherein R¹⁰ represents an alkyl radical selected from the group consisting of methyl, ethyl, n-propyl, iso-propyl, n-butyl, iso-butyl, sec-butyl and tert-butyl,
a -(C=O)-NH-R¹¹ group, wherein R¹¹ represents a phenyl group, which is mono-substituted with methyl or ethyl,
a -(C=O)-R¹² group, wherein R¹² represents a phenyl group, which is mono-substituted with -O-(C=O)-C₁₋₃-alkyl radical in the ortho position or with an -CH₂-N(C₁₋₄-alkyl)₂ in the meta or para position or with a 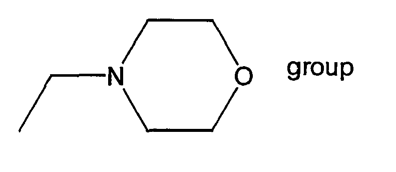 in the meta or para position.

7. Compounds according to any one of claims 1-6, **characterized in that** R⁶, R⁷ and R⁸ each represent hydrogen.

8. Compounds according to any one of claims 1 to 7, **characterized in that** A represents a -CH₂- group.

9. Compounds according to any one of claims 1 to 8, **characterized in that** a represents 1, 2 or 3, preferably 1 or 2, more preferably 1.

10. Compounds according to any one of claims 1 to 9, **characterized in that** b represents 0, 1 or 2, preferably 0 or 1.

11. Compounds according to any one of claims 1 to 10, **characterized in that** c represents 0, 1 or 2, preferably 0 or 1.

12. Compounds according to any one of claims 1 to 11, **characterized in that** at least one of the substituents R¹, R², R³ and R⁴ represents -OR⁸.

13. Compounds according to any one of claims 1 to 12, **characterized in that** one or two of the substituents R¹, R², R³ and R⁴ represent -OR⁸.

14. Compounds according to any one of claims 1 to 13, **characterized in that** one or two of the substituents R¹, R², R³ and R⁴ represent -OR⁸ and b and c each represent 0,
more peferably one of the substituents R¹, R², R³ and R⁴ represents -OR⁸ and b and c each represent 0.

15. Compounds according to any one of claims 1-14 selected from the group consisting of:
[1] 2-[4-(3-Hydroxy-2-hydroxymethyl-phenylamino)-piperidin-1-yl]-N-(9-methyl-9H-carbazol-3-yl)-acetamide,
[2] 2-[4-(4-Hydroxy-2-hydroxymethyl-phenylamino)-piperidin-1-yl]-N-(9-methyl-9H-carbazol-3-yl)-acetamide,
[3] 2-[4-(5-Hydroxy-2-hydroxymethyl-phenylamino)-piperidin-1-yl]-N-(9-methyl-9H-carbazol-3-yl)-acetamide,
[4] 2-[4-(6-Hydroxy-2-hydroxymethyl-phenylamino)-piperidin-1-yl]-N-(9-methyl-9H-carbazol-3-yl)-acetamide,
[5] 2-[4-(3-Hydroxy-2-hydroxymethyl-phenylamino)-piperidin-1-yl]-N-(9-ethyl-9H-carbazol-3-yl)-acetamide,
[6] 2-[4-(4-Hydroxy-2-hydroxymethyl-phenylamino)-piperidin-1-yl]-N-(9-ethyl-9H-carbazol-3-yl)-acetamide,
[7] 2-[4-(5-Hydroxy-2-hydroxymethyl-phenylamino)-piperidin-1-yl]-N-(9-ethyl-9H-carbazol-3-yl)-acetamide and
[8] 2-[4-(6-Hydroxy-2-hydroxymethyl-phenylamino)-piperidin-1-yl]-N-(9-ethyl-9H-carbazol-3-yl)-acetamide,
optionally in form of a salt, preferably a physiologically acceptable salt, more preferably in form of a physiologically acceptable acid addition salt, most preferably a hydrochloride salt, or a corresponding solvate.

16. Process for the preparation of 1,4-disubstituted piperidine compounds according to one or more of claims 1-15, **characterized in that** at least one compound of general formula (II), 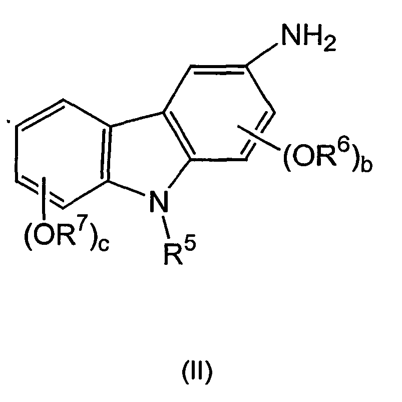 wherein R⁵, R⁶ and R⁷, b and c have the meaning according to one or more of claims 1-15; is reacted with at least one compound of general formula (III), 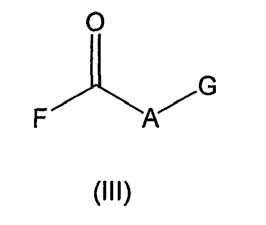 wherein A has the meaning according to one or more of claims 1-15, F represents halogen, preferably chlorine, hydroxy or an O-acyl group and G represents halogen, preferably chlorine, in a suitable reaction medium and preferably in the presence of at least one base and/or at least one auxiliary agent, and reacting the so obtained compound of general (IV) 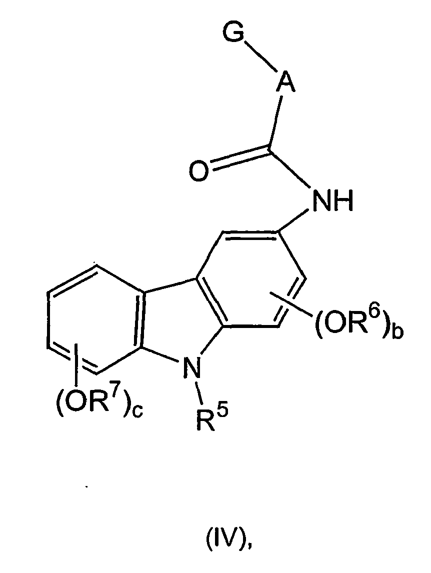 wherein A, G, R⁵, R⁶ and R⁷, b and c have the above defined meaning, with at least one piperidine compound of general formula (V) and/or a salt, preferably hydrochloride, thereof, 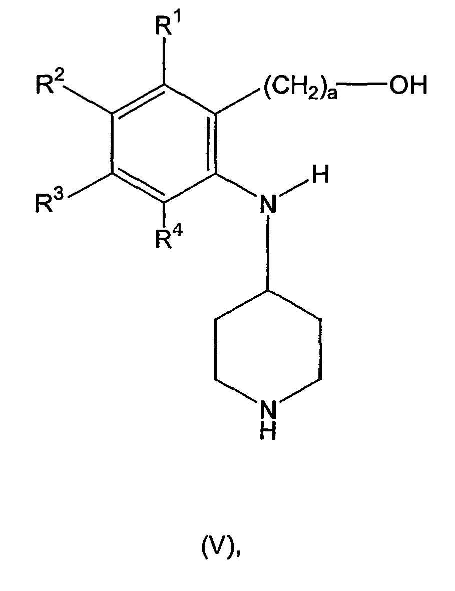 wherein R¹ to R⁴ and a have the meaning according to one or more of claims 1-15, in a suitable reaction medium, optionally in the presence of at least one base and/or at least one auxiliary agent.

17. Process for the preparation of a physiologically acceptable salt of the 1,4-disubstituted piperidine compounds according to claims 1-15, **characterized in that** at least one compound of general formula (I) is reacted with at least one acid, preferably an inorganic or organic acid, preferably in the presence of a suitable reaction medium.

18. Process for the preparation of a physiologically acceptable salt of the 1,4-disubstituted piperidine compounds according to claims 1-15, **characterized in that** at least one compound of general formula (I) having at least one acidic group is reacted with at least one base, preferably in the presence of a suitable reaction medium.

19. Medicament comprising at least one 1,4-disubstituted piperidine compound according to any one of claims 1-15 and optionally one or more pharmaceutically acceptable adjuvants.

20. Medicament according to claim 19 for the regulation of appetite, for the regulation of body weight, for the prophylaxis and/or treatment of disorders related to food ingestion, preferably selected from the group consisting of obesity, anorexia, cachexia, bulimia and/or diabetes (particularly type (II) diabetes).

21. Medicament according to claim 19 for the improvement of cognition (cognitive enhancement); for the prophylaxis and/or treatment of disorders of the peripheral nervous system; for the prophylaxis and/or treatment of disorders of the central nervous system; for the prophylaxis and/or treatment of arthritis; for the prophylaxis and/or treatment of epilepsy; for the prophylaxis and/or treatment of anxiety; for the prophylaxis and/or treatment of depression; for the prophylaxis and/or treatment of cognitive disorders, preferably memory disorders; for the prophylaxis and/or treatment of cardiovascular diseases; for the prophylaxis and/or treatment of pain; for the prophylaxis and/or treatment of hypertensive syndrom; for the prophylaxis and/or treatment of inflammatory diseases; for the prophylaxis and/or treatment of immune diseases; for the prophylaxis and/or treatment of panic attacks and/or for the prophylaxis and/or treatment of bipolar disorders.

22. Use of at least one 1,4-disubstituted piperidine compound according to any one of claims 1-15 for the manufacture of a medicament for the regulation of appetite, for the regulation of body weight, for the prophylaxis and/or treatment of disorders related to food ingestion, preferably selected from the group consisting of obesity, anorexia, cachexia, bulimia and diabetes (particularly type (II) diabetes).

23. Use of at least one 1,4-disubstituted piperidine compound according to any one of claims 1-15 for the manufacture of a medicament for the improvement of cognition (cognitive enhancement); for the prophylaxis and/or treatment of disorders of the peripheral nervous system; for the prophylaxis and/or treatment of disorders of the central nervous system; for the prophylaxis and/or treatment of arthritis; for the prophylaxis and/or treatment of epilepsy; fbr the prophylaxis and/or treatment of anxiety; for the prophylaxis and/or treatment of depression; for the prophylaxis and/or treatment of cognitive disorders, preferably memory disorders; for the prophylaxis and/or treatment of cardiovascular diseases; for the prophylaxis and/or treatment of pain; for the prophylaxis and/or treatment of hypertensive syndrom; for the prophylaxis and/or treatment of inflammatory diseases; for the prophylaxis and/or treatment of immune diseases; for the prophylaxis and/or treatment of panic attacks and for the prophylaxis and/or treatment of bipolar disorders.

## Patentansprüche

1. 1,4-disubstituierte Piperidinverbindungen der allgemeinen Formel (I), 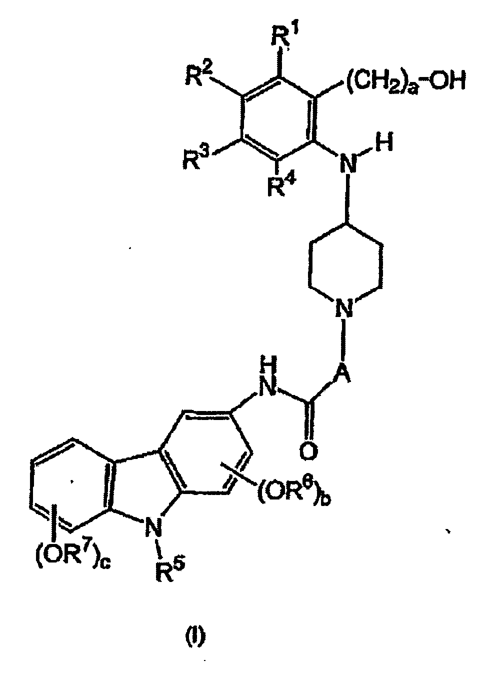 worin
a für 0, 1, 2, 3 oder 4,
b für 0, 1, 2 oder 3,
c für 0, 1, 2, 3 oder 4 steht,
R¹, R², R³, R⁴ jeweils unabhängig voneinander für einen Rest stehen, der ausgewählt ist aus der Gruppe bestehend aus Wasserstoff; Halogen; -CN; -NO₂; OR⁸; einem linearen oder verzweigten, gesättigten oder ungesättigten, ggf. wenigstens einfach substituierten aliphatischen Rest; einem gesättigten oder ungesättigten, ggf. wenigstens einfach substituierten, ggf. wenigstens ein Heteroatom als Ringglied enthaltenden cycloaliphatischen Rest, der über eine Alkylengruppe gebunden sein kann; oder einem ggf. wenigstens einfach substituierten Aryl- oder Heteroarylrest, der über eine Alkylengruppe gebunden und/oder mit einem ggf. wenigstens einfach substituierten, gesättigten oder ungesättigten, mono- oder bicyclischen Ringsystem kondensiert sein kann,
R⁵ für Wasserstoff, einen linearen oder verzweigten, gesättigten oder ungesättigten, ggf. wenigstens einfach substituierten aliphatischen Rest, oder einen gesättigten oder ungesättigten, ggf. wenigstens einfach substituierten cycloaliphatischen Rest steht,
R⁶, R⁷ und R⁸ gleich oder verschieden sind und jeweils für Wasserstoff oder einen Prodrugrest stehen,
A für eine -CH₂- oder -CH₂-CH₂-Gruppe steht,
jeweils ggf. in Form ihrer einzelnen Stereoisomeren, vorzugsweise Enantiomeren oder Diastereomeren, ihrer Racemate oder Mischungen von jeweils wenigstens zwei ihrer Stereoisomeren, vorzugsweise Enantiomeren oder Diastereomeren, in einem beliebigen Mischungsverhältnis, oder jeweils in Form ihrer Salze, bevorzugt ihrer physiologisch verträglichen Salze, oder jeweils in Form entsprechender Solvate.

2. Verbindungen gemäß Anspruch 1, **dadurch gekennzeichnet, dass** R¹, R², R³, R⁴ jeweils unabhängig voneinander für einen Rest stehen, der ausgewählt ist aus der Gruppe bestehend aus H; F; Cl; Br⁻; -CN; -NO₂; -OR⁸; einem linearen oder verzweigten, gesättigten oder ungesättigten, ggf. wenigstens einfach substituierten, aliphatischen Rest mit 1 bis 6 C-Atomen; einem gesättigten oder ungesättigten, ggf. wenigstens einfach substituierten, ggf. wenigstens ein Heteroatom als Ringglied enthaltenden cycloaliphatischen Rest mit 3 bis 8 C-Atomen, der über eine C₁₋₃-Alkylengruppe gebunden sein kann, oder einem ggf. wenigstens einfach substituierten Aryl- oder Heteroarylrest, der über eine C₁₋₃-Alkylengruppe gebunden und/oder mit einem ggf. wenigstens einfach substituierten, gesättigten oder ungesättigten, mono- oder bicyclischen Ringsystem kondensiert sein kann,
R⁵ für Wasserstoff, einen linearen oder verzweigten, gesättigten oder ungesättigten, ggf. wenigstens einfach substituierten aliphatischen Rest mit 1 bis 6 C-Atomen oder einen gesättigten oder ungesättigten, ggf. wenigstens einfach substituierten, cycloaliphatischen Rest mit 3 bis 8 C-Atomen steht,
R⁶, R⁷ und R⁸ gleich oder verschieden sind und jeweils für Wasserstoff oder einen Prodrugrest stehen, und
A für eine -CH₂- oder -CH₂-CH₂-Gruppe steht.

3. Verbindungen gemäß Anspruch 1 oder 2, **dadurch gekennzeichnet, dass** R¹, R², R³, R⁴ jeweils unabhängig voneinander für einen Resr steht, der ausgewählt ist aus der Gruppe bestehend aus H; F; Cl; Br⁻; -CN; -NO₂; -OR⁸; einem linearen oder verzweigten, ggf. wenigstens einfach substituierten C₁₋₄ Alkyl-Rest, einem gesättigten, ggf. wenigstens einfach substituierten, ggf. wenigstens ein Heteroatom als Ringglied enthaltenden, cycloaliphatischen Rest mit 5 oder 6 C-Atomen, der über eine ggf. wenigstens einfach substituierte C₁- oder C₂-Alkylengruppe gebunden sein kann,
wobei R¹, R², R³, R⁴ jeweils unabhängig voneinander bevorzugt aus der Gruppe bestehend aus H; F; Cl; Br; -CN; -NO₂; -CH₃; -CH₂-CH₃; -CHF₂; -CH₂F; -CF₃; -CF₂CF₃; -OR⁸; Cyclopentyl und Cyclohexyl, besonders bevorzugt aus der Gruppe bestehend aus H; F; Cl; Br; -CH₃ and -OR⁸ ausgewählt sind.

4. Verbindungen gemäß einem der Ansprüche 1 bis 3, **dadurch gekennzeichnet, dass** R⁵ für H oder einen linearen oder verzweigten C₁₋₆-Alkylrest steht,
wobei R⁵ vorzugsweise für H oder für einen Alkylrest steht, der ausgewählt ist aus der Gruppe bestehend aus Methyl, Ethyl, n-Propyl, iso-Propyl, n-Butyl, iso-Butyl, sec-Butyl und tert-Butyl.

5. Verbindungen gemäß einem der Ansprüche 1 bis 4, **dadurch gekennzeichnet, dass** R⁶, R⁷ und R⁸ gleich oder verschieden sind und jeweils für H oder für einen Prodrugrest stehen, der ausgewählt ist aus der Gruppe bestehend aus
einem linearen oder verzweigten C₁₋₃-Alkylrest,
einer P(=O)(OR⁹)₂-Gruppe, worin R⁹ für einen linearen oder verzweigten C₁₋₄-Alkylrest steht,
einer -(C=O)-O-R¹⁰-Gruppe, worin R¹⁰ für einen linearen oder verzweigten C₁₋₅-Alkylrest steht,
einer -(C=O)-NH-R¹¹-Gruppe, worin R¹¹ für eine durch einen linearen oder verzweigten C₁₋₃-Alkylrest einfach substituierte Phenylgruppe steht,
einer -(C=O)-R¹²-Gruppe, worin R¹² für eine durch einen -O-(C=O)-C₁₋₃-Alkylrest, eine -CH₂-N(C₁₋₄-Alkyl)₂-Gruppe oder eine Gruppe der Formel 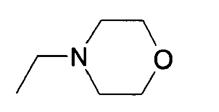 einfach substituierte Phenylgruppe steht.

6. Verbindungen gemäß Anspruch 5, **dadurch gekennzeichnet, dass** R⁶, R⁷ und R⁸ gleich oder verschieden sind und jeweils für H oder für einen Prodrugrest stehen, der ausgewählt ist aus Gruppe bestehend aus
einem linearen oder verzweigten Alkylrest, der ausgewählt ist aus der Gruppe bestehend aus Methyl, Ethyl, n-Propyl und iso-Propyl,
einer P(=O)(OR⁹)₂ Gruppe, worin R⁹ für Methyl oder Ethyl steht,
einer -(C=O)-O-R¹⁰-Gruppe, worin R¹⁰ für einen Alkylrest steht, der ausgewählt ist aus der Gruppe bestehend aus Methyl, Ethyl, n-Propyl, iso-Propyl, n-Butyl, iso-Butyl, sec-Butyl und tert-Butyl,
einer -(C=O)-NH-R¹¹-Gruppe, worin R¹¹ für eine durch Methyl oder Ethyl einfach substituierte Phenylgruppe steht,
einer -(C=O)-R¹² Gruppe, worin R¹² für eine durch einen -O-(C=O)-C₁₋₃-Alkylrest in ortho-Stellung oder eine -CH₂-N(C₁₋₄-Alkyl)₂ Gruppe in metha- oder para-Stellung oder einer Gruppe der Formel 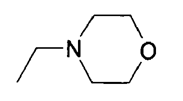 in metha- oder para-Stellung monosubstituierte Phenylgruppe steht.

7. Verbindungen gemäß einem der Ansprüche 1 bis 6, **dadurch gekennzeichnet, dass** R⁶, R⁷ und R⁸ jeweils für Wasserstoff stehen.

8. Verbindungen gemäß einem der Ansprüche 1 bis 7, **dadurch gekennzeichnet, dass** A für eine -CH₂-Gruppe steht.

9. Verbindungen gemäß einem der Ansprüche 1 bis 8, **dadurch gekennzeichnet, dass** a für 1, 2, oder 3, bevorzugt für 1 oder 2, besonders bevorzugt für 1 steht.

10. Verbindungen gemäß einem der Ansprüche 1 bis 9, **dadurch gekennzeichnet, dass** b für 0, 1 oder 2, bevorzugt für 0 oder 1 steht.

11. Verbindungen gemäß einem der Ansprüche 1 bis 10, **dadurch gekennzeichnet, dass** c für 0, 1 oder 2, bevorzugt für 0 oder 1 steht.

12. Verbindungen gemäß einem der Ansprüche 1 bis 11, **dadurch gekennzeichnet, dass** wenigstens einer der Substituenten R¹, R², R³ und R⁴ für -OR⁸ steht.

13. Verbindungen gemäß einem der Ansprüche 1 bis 12, **dadurch gekennzeichnet, dass** einer oder zwei der Substituenten R¹, R², R³, R⁴ für -OR⁸ stehen.

14. Verbindungen gemäß einem der Ansprüche 1 bis 13, **dadurch gekennzeichnet, dass** einer oder zwei der Substituenten R¹, R², R³, R⁴ für -OR⁸ und b und c jeweils für 0 stehen,
bevorzugt einer der Substituenten R¹, R², R³, R⁴ für -OR⁸ steht und b und c jeweils für 0 stehen.

15. Verbindungen gemäß einem der Ansprüche 1 bis 14, ausgewählt aus der Gruppe bestehend aus:
[1] 2-[4-(3-Hydroxy-2-hydroxymethylphenylamino)piperidin-1-yl]-N-(9-methyl-9H-carbazol-3-yl)acetamid,
[2] 2-[4-(4-Hydroxy-2-hydroxymethylphenylamino)piperidin-1-yl]-N-(9-methyl-9H-carbazol-3-yl)acetamid,
[3] 2-[4-(5-Hydroxy-2-hydroxymethylphenylamino)piperidin-1-yl]-N-(9-methyl-9H-carbazol-3-yl)acetamid,
[4] 2-[4-(6-Hydroxy-2-hydroxymethylphenylamino)piperidin-1-yl]-N-(9-methyl-9H-carbazol-3-yl)acetamid,
[5] 2-[4-(3-Hydroxy-2-hydroxymethylphenylamino)piperidin-1-yl]-N-(9-ethyl-9H-carbazol-3-yl)acetamid,
[6] 2-[4-(4-Hydroxy-2-hydroxymethylphenylamino)piperidin-1-yl]-N-(9-ethyl-9H-carbazol-3-yl)acetamid,
[7] 2-[4-(5-Hydroxy-2-hydroxymethylphenylamino)piperidin-1-yl]-N-(9-ethyl-9H-carbazol-3-yl)acetamid und
[8] 2-[4-(6-Hydroxy-2-hydroxymethylphenylamino)piperidin-1-yl]-N-(9-ethyl-9H-carbazol-3-yl)acetamid,
jeweils ggf. in Form von ihren Salzen, bevorzugt physiologisch verträglichen Salzen, besonders bevorzugt physiologisch verträglichen Säureadditionssalzen, ganz besonders bevorzugt in Form ihrer Chlorhydrate, oder jeweils in Form der entsprechenden Solvate.

16. Verfahren zur Herstellung von 1,4-disubstituierter Piperidinverbindungen gemäß einem oder mehreren der Ansprüche 1 bis 15, **dadurch gekennzeichnet, dass** wenigstens eine Verbindung der Formel (II), 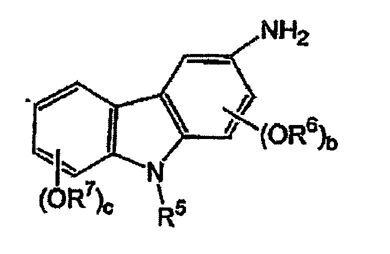 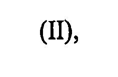 worin R⁵, R⁶ und R⁷, b und c jeweils die Bedeutungen gemäß einem oder mehreren der Ansprüche 1 bis 15 haben, mit wenigstens einer Verbindung der allgemeinen Formel (III), 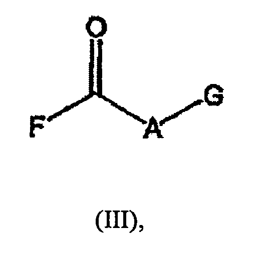 worin A die Bedeutung gemäß einem oder mehreren der Ansprüche 1 bis 15 hat, F für Halogen, vorzugsweise Chlor, für Hydroxy oder für eine O-Acylgruppe steht und G für Halogen, vorzugsweise Chlor steht, in einem geeigneten Reaktionsmedium und vorzugsweise in Gegenwart von wenigstens einer Base und/oder wenigstens einem Hilfsmittel umgesetzt wird und die so erhaltene Verbindung der allgemeinen Formel (IV) 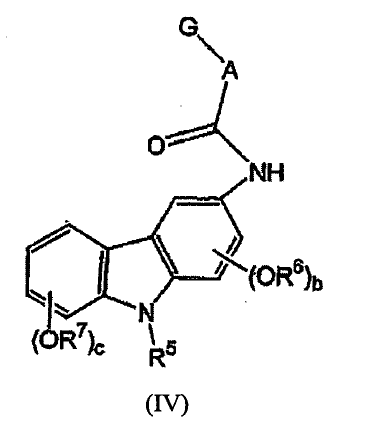 worin A, G, R⁵, R⁶ und R⁷, b und c die jeweils obenstehend definierten Bedeutungen haben, mit jeweils wenigstens einer Piperidinverbindung der allgemeinen Formel (V) 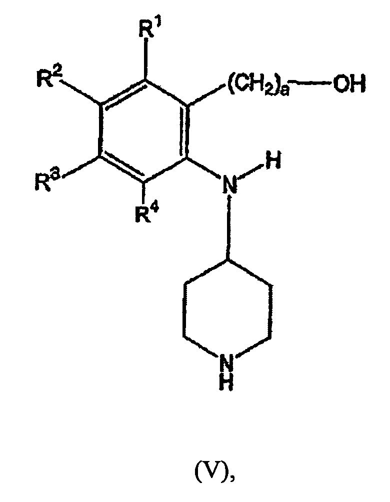 worin R¹ bis R⁴ und a jeweils die Bedeutung gemäß einem der Ansprüche 1 bis 15 haben, und/oder einem ihrer Salze, vorzugsweise dem Chlorhydrat, in einem geeigneten Reaktionsmedium ggf. in Gegenwart von wenigstens einer Base und/oder wenigstens einem Hilfsmittel umgesetzt wird.

17. Verfahren zur Herstellung von physiologisch verträglichen Salzen der 1,4-disubstituierten Piperidinverbindungen gemäß den Ansprüchen 1 bis 15, **dadurch gekennzeichnet, dass** wenigstens eine Verbindung der allgemeinen Formel (I) mit wenigstens einer Säure, vorzugsweise einer anorganischen oder organischen Säure, bevorzugt in Gegenwart von einem geeigneten Reaktionsmedium umgesetzt wird.

18. Verfahren zur Herstellung physiologisch verträglicher Salze der 1,4-disubstituierten Piperidinverbindungen gemäß den Ansprüchen 1 bis 15, **dadurch gekennzeichnet, dass** wenigstens eine wenigstens eine saure Gruppe enthaltende Verbindung der allgemeinen Formel (I) mit wenigstens einer Base vorzugsweise in Gegenwart von einem geeigneten Reaktionsmedium umgesetzt wird.

19. Arzneimittel umfassend wenigstens eine 1,4-disubstituierte Piperidinverbindung gemäß einem der Ansprüche 1 bis 15 und ggf. ein oder mehrere pharmazeutisch verträgliche Hilfsmittel.

20. Arzneimittel gemäß Anspruch 19 zur Appetitregelung, zur Regelung des Körpergewichts, zur Prophylaxe und/oder Behandlung von Störungen der Nahrungsaufnahme, vorzugsweise ausgewählt aus der Gruppe bestehend aus Fettsucht, Anorexie, Kaschexie, weise ausgewählt aus der Gruppe bestehend aus Fettsucht, Anorexie, Kaschexie, Bulimie und/oder Diabetes mellitus (insbesondere Diabetes mellitus Typ 2).

21. Arzneimittel gemäß Anspruch 19 zur Verbesserung des Erkenntnisvermögens (Steigerung der Erkenntnisleistung); zur Prophylaxe und/oder Behandlung von Störungen des peripheren Nervensystems; zur Prophylaxe und/oder Behandlung von Störungen des Zentralnervensystems; zur Prophylaxe und/oder Behandlung von Arthritis; zur Prophylaxe und/oder Behandlung von Epilepsie; zur Prophylaxe und/oder Behandlung von Angstzuständen; zur Prophylaxe und/oder Behandlung von Depressionen; zur Prophylaxe und/oder Behandlung von Erkenntnisstörungen, insbesondere Gedächtnisstörungen; zur Prophylaxe und/oder Behandlung von kardiovaskulären Krankheiten; zur Prophylaxe und/oder Behandlung von Schmerzen; zur Prophylaxe und/oder Behandlung des hypertensiven Syndroms, zur Prophylaxe und/oder Behandlung von Entzündungskrankheiten; zur Prophylaxe und/oder Behandlung von Immunkrankheiten; zur Prophylaxe und/oder Behandlung von Panikanfällen und/oder zur Prophylaxe und/oder Behandlung bipolarer Störungen.

22. Verwendung wenigstens einer 1,4-disubstituierten Piperidinverbindung gemäß einem der Ansprüche 1 bis 15 zur Herstellung eines Arzneimittels zur Appetitregelung, zur Regelung des Körpergewichts und/oder zur Prophylaxe und/oder Behandlung von Störungen der Nahrungsaufnahme, vorzugsweise ausgewählt aus der Gruppe bestehend aus Fettsucht, Anorexie, Kachexie, Bulimie und Diabetes mellitus (insbesondere Diabetes mellitus Typ 2).

23. Verwendung wenigstens einer 1,4-disubstituierten Piperidinverbindung gemäß einem der Ansprüche 1 bis 15 zur Herstellung eines Arzneimittels zur Verbesserung des Erkenntnisvermögens (Steigerung der Erkenntnisleistung); zur Prophylaxe und/oder Behandlung von Störungen des peripheren Nervensystems; zur Prophylaxe und/oder Behandlung von Störungen des Zentralnervensystems; zur Prophylaxe und/oder Behandlung von Arthritis; zur Prophylaxe und/oder Behandlung von Epilepsie; zur Prophylaxe und/oder Behandlung von Angstzuständen; zur Prophylaxe und/oder Behandlung von Depressionen; zur Prophylaxe und/oder Behandlung von Erkenntnisstörungen, insbesondere Gedächtnisstörungen; zur Prophylaxe und/oder Behandlung von kardiovaskulären Krankheiten; zur Prophylaxe und/oder Behandlung von Schmerzen; zur Prophylaxe und/oder Behandlung des hypertensiven Syndroms, zur Prophylaxe und/oder Behandlung von Entzündungskrankheiten; zur Prophylaxe und/oder Behandlung von krankheiten; zur Prophylaxe und/oder Behandlung von Immunkrankheiten; zur Prophylaxe und/oder Behandlung von Panikanfällen und/oder zur Prophylaxe und/oder Behandlung bipolarer Störungen.

## Revendications

1. Composés de pipéridine 1,4-disubstitués de formule générale (I), 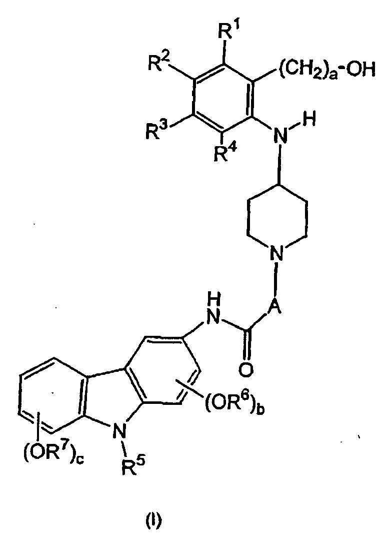 dans laquelle
a représente la valeur 0, 1, 2, 3 ou 4,
b représente la valeur 0, 1, 2 ou 3,
c représente la valeur 0, 1, 2, 3 ou 4,
R¹, R², R³, R⁴ sont chacun indépendamment choisis dans le groupe composé de l'hydrogène ; d'un halogène ; d'un groupe -CN ; d'un groupe -NO₂; d'un groupe -OR⁸; d'un radical aliphatique linéaire ou ramifié, saturé ou insaturé, de façon facultative au moins monosubstitué ; d'un radical cycloaliphatique saturé ou insaturé, de façon facultative au moins monosubstitué, contenant de façon facultative au moins un hétéroatome en tant qu'élément cyclique, qui peut être lié via un groupe alkylène ; ou un radical aryle ou hétéroaryle de façon facultative au moins monosubstitué, qui peut être lié via un groupe alkylène et/ou qui peut être condensé avec un système d'anneau mono ou bicyclique de façon facultative au moins monosubstitué, saturé ou insaturé.
R⁵ représente l'hydrogène, un radical aliphatique linéaire ou ramifié, saturé ou insaturé, de façon facultative au moins monosubstitué, ou un radical cycloaliphatique saturé ou insaturé, de façon facultative au moins monosubstitué.
R⁶, R⁷ et R⁸, identiques ou différents, représentent chacun l'hydrogène ou un groupe caractéristique de promédicament,
A représente un groupe -CH₂- ou -CH₂-CH₂-,
de façon facultative sous la forme de l'un de ses stéréoisomères, de préférence ses énantiomères ou ses diastéréoisomères, de son racémate ou sous la forme d'un mélange d'au moins deux de ses stéréoisomères, de préférence ses énantiomères ou ses diastéréoisomères, dans n'importe quel rapport de mélange, ou d'un sel, de préférence un sel acceptable sur le plan physiologique de celui-ci, ou d'un solvate correspondant, respectivement.

2. Composés selon la revendication 1, **caractérisés en ce que** R¹, R², R³, R⁴; sont chacun indépendamment choisis dans le groupe composé de l'hydrogène ; du fluor ; du chlore ; du brome ; du groupe -CN ; du groupe -NO₂ ; du groupe -OR⁸; d'un radical aliphatique en C₁ à C₆ linéaire ou ramifié, saturé ou insaturé, de façon facultative au moins monosubstitué ; d'un radical cycloaliphatique en C₃ à C₈ saturé ou insaturé, de façon facultative au moins monosubstitué, contenant de façon facultative au moins un hétéroatome en tant qu'élément cyclique, qui peut être lié via un groupe alkylène en C₁ à C₃ ; ou d'un radical aryle ou hétéroaryle de façon facultative au moins monosubstitué, qui peut être lié via un groupe alkylène en C₁ à C₃ et/ou qui peut être condensé avec un système d'anneau mono ou bicyclique de façon facultative au moins monosubstitué, saturé ou insaturé,
R⁵ représente l'hydrogène, un radical aliphatique en C₁ à C₆ linéaire ou ramifié, saturé ou insaturé, de façon facultative au moins monosubstitué, ou un radical cycloaliphatique en C₃ à C₈ saturé ou insaturé, de façon facultative au moins monosubstitué.
R⁶, R⁷ et R⁸, identiques ou différents, représentent chacun l'hydrogène ou un groupe caractéristique de promédicament,
A représente un groupe -CH₂- ou un groupe -CH₂-CH₂-.

3. Composés selon la revendication 1 ou 2, **caractérisés en ce que** R¹, R², R³, R⁴ sont chacun indépendamment choisis dans le groupe composé de l'hydrogène ; du fluor ; du chlore ; du brome ; du groupe -CN ; du groupe -NO₂ ; du groupe -OR⁸ ; d'un radical alkyle en C₁ à C₄ linéaire ou ramifié, de façon facultative au moins monosubstitué ; d'un radical cycloaliphatique en C₅ ou en C₆ saturé, de façon facultative au moins monosubstitué, contenant de façon facultative au moins un hétéroatome en tant qu'élément cyclique, qui peut être lié via un groupe alkylène en C₁ ou en C₂ de façon facultative au moins monosubstitué ;
de préférence R¹, R², R³, R⁴ sont chacun indépendamment choisis dans le groupe composé de l'hydrogène ; du fluor ; du chlore ; du brome ; du groupe -CN ; du groupe -NO₂; du groupe -CH₃ ; du groupe -CH₂CH₃ ; du groupe -CHF₂ ; du groupe -CH₂F ; du groupe -CF₃ ; du groupe -CF₂CF₃ ; du groupe OR⁸; du groupe cyclopentyle et du groupe cyclohexyle,
de façon plus préférée R¹, R², R³, R⁴ sont chacun indépendamment choisis dans le groupe composé de l'hydrogène ; du fluor ; du chlore ; du brome ; du groupe CH₃ et du groupe OR⁸.

4. Composés selon l'une quelconque des revendications 1 à 3, **caractérisés en ce que** R⁵ représente l'hydrogène ou un radical alkyle en C₁ à C₆ linéaire ou ramifié,
de préférence R⁵ représente l'hydrogène ou un radical alkyle choisi dans le groupe composé d'un groupe méthyle, d'un groupe éthyle, d'un groupe n-propyle, d'un groupe isopropyle ; d'un groupe n-butyle, d'un groupe isobutyle, d'un groupe sec-butyle et d'un groupe tert-butyle.

5. Composés selon l'une quelconque des revendications 1 à 4, **caractérisés en ce que** R⁶, R⁷ et R⁸, identiques ou différents, représentent chacun l'hydrogène ou un groupe caractéristique de promédicament choisi dans le groupe composé
d'un groupe alkyle en C₁ à C₃ linéaire ou ramifié,
d' un groupe P(=O) (OR⁹)₂, dans lequel R⁹ représente un radical alkyle en C₁ à C₄ linéaire ou ramifié,
d'un groupe -(C=O)-O-R¹⁰, dans lequel R¹⁰ représente un radical alkyle en C₁ à C₅ linéaire ou ramifié,
d'un groupe -(C=O)-NH-R¹¹, dans lequel R¹¹ représente un groupe phényle, qui est monosubstitué avec un radical alkyle en C₁ à C₃ linéaire ou ramifié,
d'un groupe -(C=O) -R¹², dans lequel R¹² représente un groupe phényle, qui est monosubstitué avec un radical O-(C=O)-alkyle en C₁ à C₃, un groupe -CH₂-N(alkyle en C₁ à C₄) ou un groupe

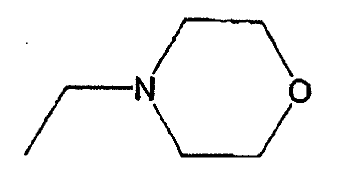

6. Composés selon la revendication 5, **caractérisés en ce que** R⁶, R⁷ et R⁸, identiques ou différents, représentent chacun l'hydrogène ou un groupe caractéristique de promédicament choisi dans le groupe composé
d'un groupe alkyle linéaire ou ramifié choisi dans le groupe composé d'un groupe méthyle, d'un groupe éthyle, d'un groupe n-propyle et d'un groupe isopropyle,
d'un groupe P (=O) (OR⁹)₂, dans lequel R⁹ représente un groupe méthyle ou un groupe éthyle,
d'un groupe -(C=O)-O-R¹⁰, dans lequel R¹⁰ représente un radical alkyle choisi dans le groupe composé d'un groupe méthyle, d'un groupe éthyle, d'un groupe n-propyle, d'un groupe isopropyle, d'un groupe n-butyle, d'un groupe isobutyle, d'un groupe sec-butyle et d'un groupe tert-butyle,
d'un groupe -(C=O)-NH-R¹¹, dans lequel R¹¹ représente un groupe phényle, qui est monosubstitué avec un groupe méthyle ou un groupe éthyle,
d' un groupe -(C=O)-R¹², dans lequel R¹² représente un groupe phényle, qui est monosubstitué avec un radical -O-(C=O)-alkyle en C₁ à C₃ dans la position ortho ou avec un groupe -CH₂-N(alkyle en C₁ à C₄)₂ dans la position méta ou para ou avec un groupe 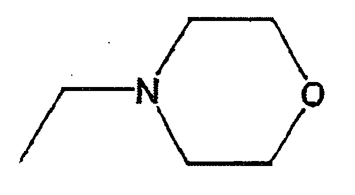 dans la position méta ou para.

7. Composés selon l'une quelconque des revendications 1 à 6, **caractérisés en ce que** R⁶, R⁷ et R⁸ représentent chacun l'hydrogène.

8. Composés selon l'une quelconque des revendications 1 à 7, **caractérisés en ce que** A représente un groupe -CH₂-.

9. Composés selon l'une quelconque des revendications 1 à 8, **caractérisés en ce que** a représente la valeur 1, 2 ou 3, de préférence la valeur 1 ou 2, de façon plus préférée la valeur 1.

10. Composés selon l'une quelconque des revendications 1 à 9, **caractérisés en ce que** b représente la valeur 0, 1 ou 2, de préférence la valeur 0 ou 1.

11. Composés selon l'une quelconque des revendications 1 à 10, **caractérisés en ce que** c représente la valeur 0, 1 ou 2, de préférence la valeur 0 ou 1.

12. Composés selon l'une quelconque des revendications 1 à 11, **caractérisés en ce que** au moins un des substituants R¹, R², R³ et R⁴ représente le groupe -OR⁸.

13. Composés selon l'une quelconque des revendications 1 à 12, **caractérisés en ce que** un ou deux des substituants R¹, R², R³ et R⁴ représentent le groupe -OR⁸.

14. Composés selon l'une quelconque des revendications 1 à 13, **caractérisés en ce que** un ou deux des substituants R¹, R², R³ et R⁴ représentent un groupe -OR⁸ et b et c représentent chacun la valeur 0,
de façon plus préférée, un des substituants R¹, R², R³ et R⁴ représente le groupe -OR⁸ et b et c représentent chacun la valeur 0.

15. Composés selon l'une quelconque des revendications 1 à 14, choisis dans le groupe composé de :
[1] 2-[4-(3-hydroxy-2-hydroxyméthyl-phénylamino)-pipéridin-1-yl]-N-(9-méthyl-9H-carbazol-3-yl)-acétamide,
[2] 2-[4-(4-hydroxy-2-hydroxyméthyl-phénylamino)-pipéridin-1-yl]-N-(9-méthyl-9H-carbazol-3-yl)-acétamide,
[3] 2-[4-(5-hydroxy-2-hydroxyméthyl-phénylamino)-pipéridin-1-yl]-N-(9-méthyl-9H-carbazol-3-yl)-acétamide,
[4] 2-[4-(6-hydroxy-2-hydroxyméthyl-phénylamino)-pipéridin-1-yl]-N-(9-méthyl-9H-carbazol-3-yl)-acétamide,
[5] 2-[4-(3-hydroxy-2-hydroxyméthyl-phénylamino)-pipéridin-1-yl]-N-(9-éthyl-9H-carbazol-3-yl)-acétamide,
[6] 2-[4-(4-hydroxy-2-hydroxyméthyl-phénylamino)-pipéridin-1-yl]-N-(9-éthyl-9H-carbazol-3-yl)-acétamide,
[7] 2-[4-(5-hydroxy-2-hydroxyméthyl-phénylamino)-pipéridin-1-yl]-N-(9-éthyl-9H-carbazol-3-yl)-acétamide et
[8] 2-[4-(6-hydroxy-2-hydroxyméthyl-phénylamino)-pipéridin-1-yl]-N-(9-éthyl-9H-carbazol-3-yl)-acétamide,
de façon facultative sous la forme d'un sel, de préférence d'un sel acceptable sur le plan physiologique, de façon plus préférée sous la forme d'un sel d'addition d'acide acceptable sur le plan physiologique, de façon la plus préférée d'un sel de chlorhydrate, ou d'un solvate correspondant.

16. Procédé pour la préparation de composés de pipéridine 1,4-disubstitués selon l'une ou plusieurs des revendications 1 à 15, **caractérisé en ce que** au moins un composé de formule générale (II) 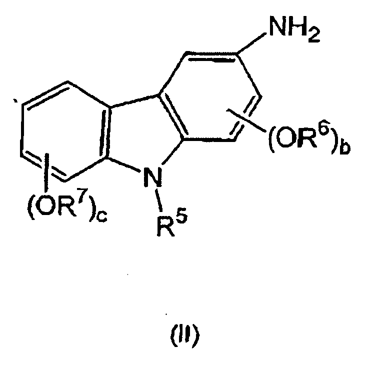 dans laquelle R⁵, R⁶ et R⁷, b et c ont la signification selon l'une ou plusieurs des revendications 1 à 15 ; est mis à réagir avec au moins un composé de formule générale (III), 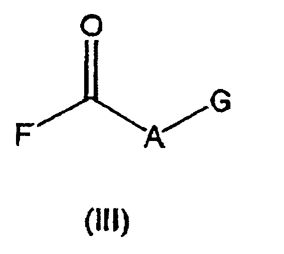 dans laquelle A a la signification selon l'une ou plusieurs des revendications 1 à 15, F représente un halogène, de préférence le chlore, un groupe hydroxy ou un groupe 0-acyle et G représente un halogène, de préférence le chlore, dans un milieu de réaction adéquat et de préférence en présence d'au moins une base et/ou d'au moins un agent auxiliaire, et en mettant à réagir le composé ainsi obtenu de formule générale (IV) 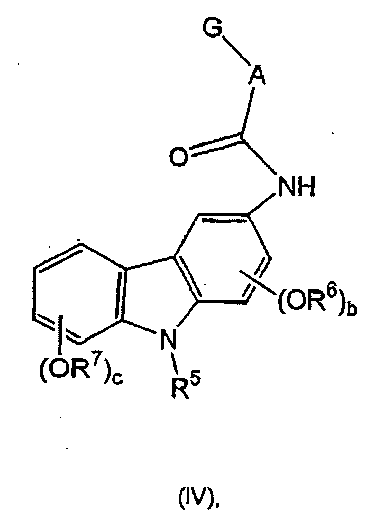 dans laquelle A, G, R⁵, R⁶ et R⁷, b et c ont la signification définie ci-dessus, avec au moins un composé de pipéridine de formule générale (V) et/ou un sel, de préférence le chlorhydrate, de celui-ci 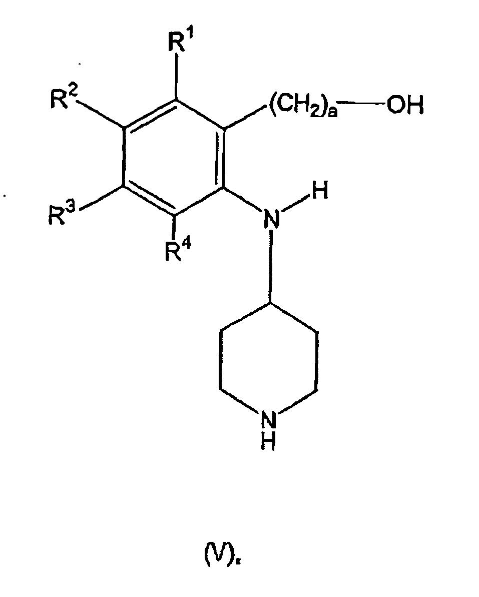 dans laquelle R¹ à R⁴ et a ont la signification selon l'une ou plusieurs des revendications 1 à 15, dans un milieu de réaction adéquat, de façon facultative en présence d'au moins une base et/ou d'au moins un agent auxiliaire.

17. Procédé pour la préparation d'un sel acceptable sur le plan physiologique de composés de pipéridine 1,4-disubstitués selon les revendications 1 à 15, **caractérisé en ce que** au moins un composé de formule générale (I) est mis à réagir avec au moins un acide, de préférence un acide inorganique ou organique, de préférence en présence d'un milieu de réaction adéquat.

18. Procédé pour la préparation d'un sel acceptable sur le plan physiologique de composés de pipéridine 1,4-disubstitués selon les revendications 1 à 15, **caractérisé en ce que** au moins un composé de formule générale (I) ayant au moins un groupe acide est mis à réagir avec au moins une base, de préférence en présence d'un milieu de réaction adéquat.

19. Médicament comprenant au moins un composé de pipéridine 1,4-disubstitué selon l'une quelconque des revendications 1 à 15 et de façon facultative un ou plusieurs adjuvants acceptables sur le plan pharmaceutique.

20. Médicament selon la revendication 19, pour la régulation de l'appétit, pour la régulation du poids corporel, pour la prophylaxie et/ou le traitement des troubles apparentés à l'ingestion de nourriture, de préférence choisis dans le groupe composé de l'obésité, de l'anorexie, de la cachexie, de la boulimie et/ou du diabète (en particulier du diabète de type (II)).

21. Médicament selon la revendication 19, pour l'amélioration de la connaissance (amélioration cognitive) ; pour la prophylaxie et/ou le traitement des troubles du système nerveux périphérique ; pour la prophylaxie et/ou le traitement des troubles du système nerveux central ; pour la prophylaxie et/ou le traitement de l'arthrite ; pour la prophylaxie et/ou le traitement de l'épilepsie ; pour la prophylaxie et/ou le traitement de l'anxiété ; pour la prophylaxie et/ou le traitement de la dépression ; pour la prophylaxie et/ou le traitement des troubles cognitifs, de préférence les troubles de la mémoire ; pour la prophylaxie et/ou le traitement des maladies cardiovasculaires ; pour la prophylaxie et/ou le traitement de la douleur ; pour la prophylaxie et/ou le traitement du syndrome de l'hypertension ; pour la prophylaxie et/ou le traitement des maladies inflammatoires ; pour la prophylaxie et/ou le traitement des maladies immunitaires ; pour la prophylaxie et/ou le traitement des crises de panique et/ou pour la prophylaxie et/ou le traitement des troubles bipolaires.

22. Utilisation d'au moins un composé de pipéridine 1,4-disubstitué selon l'une quelconque des revendications 1 à 15, pour la fabrication d'un médicament pour la régulation de l'appétit, pour la régulation du poids corporel, pour la prophylaxie et/ou le traitement des troubles apparentés à l'ingestion de nourriture, de préférence choisis dans le groupe composé de l'obésité, de l'anorexie, de la cachexie, de la boulimie et du diabète (en particulier du diabète de type (II)).

23. Utilisation d'au moins un composé de pipéridine 1,4-disubstitué selon l'une quelconque des revendications 1 à 15, pour la fabrication d'un médicament pour l'amélioration de la connaissance (amélioration cognitive) ; pour la prophylaxie et/ou le traitement des troubles du système nerveux périphérique ; pour la prophylaxie et/ou le traitement des troubles du système nerveux central ; pour la prophylaxie et/ou le traitement de l'arthrite ; pour la prophylaxie et/ou le traitement de l'épilepsie; pour la prophylaxie et/ou le traitement de l'anxiété ; pour la prophylaxie et/ou le traitement de la dépression ; pour la prophylaxie et/ou le traitement des troubles cognitifs, de préférence les troubles de la mémoire ; pour la prophylaxie et/ou le traitement des maladies cardiovasculaires ; pour la prophylaxie et/ou le traitement de la douleur ; pour la prophylaxie et/ou le traitement du syndrome de l'hypertension ; pour la prophylaxie et/ou le traitement des maladies inflammatoires ; pour la prophylaxie et/ou le traitement des maladies immunitaires ; pour la prophylaxie et/ou le traitement des crises de panique et pour la prophylaxie et/ou le traitement des troubles bipolaires.
